(19) Europäisches Patentamt
European Patent Office
Office européen des brevets

(11) EP 1 071 670 B1

(12) EUROPÄISCHE PATENTSCHRIFT

(45) Veröffentlichungstag und Bekanntmachung des Hinweises auf die Patenterteilung:
**07.07.2004 Patentblatt 2004/28**

(21) Anmeldenummer: **99920661.8**

(22) Anmeldetag: **14.04.1999**

(51) Int Cl.[7]: **C07D 249/08**, C07D 263/32, C07D 263/38, C07D 233/54, C07D 233/70, C07D 233/78, C07D 271/10, C07D 207/32, C07D 207/44, C07D 231/22

(86) Internationale Anmeldenummer:
**PCT/EP1999/002496**

(87) Internationale Veröffentlichungsnummer:
**WO 1999/054314 (28.10.1999 Gazette 1999/43)**

(54) **DIPHENYL-SUBSTITUIERTE 5-RING-HETEROCYCLEN, VERFAHREN ZU IHRER HERSTELLUNG SOWIE DEREN VERWENDUNG ALS ARZNEIMITTEL**

NOVEL DIPHENYL-SUBSTITUTED 5-RING-HETEROCYCLES, METHOD FOR PRODUCING THEM AND THEIR USE AS MEDICAMENTS

NOUVEAUX HETEROCYCLES A 5 NOYAUX A SUBSTITUTION DIPHENYLE, LEUR PROCEDE DE PREPARATION ET LEUR UTILISATION COMME MEDICAMENTS

(84) Benannte Vertragsstaaten:
**AT BE CH CY DE DK ES FI FR GB GR IE IT LI LU MC NL PT SE**

(30) Priorität: **17.04.1998 DE 19816880**
**17.04.1998 IT MI980818**

(43) Veröffentlichungstag der Anmeldung:
**31.01.2001 Patentblatt 2001/05**

(73) Patentinhaber:
- **Boehringer Ingelheim Pharma GmbH & Co.KG 55218 Ingelheim am Rhein (DE)**
- **BOEHRINGER INGELHEIM ITALIA S.p.A. 20139 Milano (IT)**

(72) Erfinder:
- **BRENNER, Michael D-55411 Bingen am Rhein (DE)**
- **BECHTEL, Wolf-Dietrich D-55437 Appenheim (DE)**
- **PALLUK, Rainer D-55411 Bingen (DE)**
- **WIENRICH, Marion D-64331 Weiterstadt (DE)**
- **WEISER, Thomas D-55268 Nieder-Olm (DE)**
- **CEREDA, Enzo I-15067 Novi Ligure (IT)**
- **BIGNOTTI, Maura I-20135 Milano (IT)**
- **PELLEGRINI, Carlomaria I-26841 Casalpusterlengo (IT)**

(74) Vertreter: **Laudien, Dieter, Dr. Boehringer Ingelheim GmbH, B Patent 55216 Ingelheim am Rhein (DE)**

(56) Entgegenhaltungen:
**EP-A- 0 248 594**
**EP-A- 0 513 387**
**WO-A-98/15274**
**WO-A-98/17652**
**WO-A-98/27108**
**DE-A- 19 528 189**
**FR-A- 2 152 345**

- **C P TAYLOR ET AL: "TRENDS IN PHARMACOLOGICAL SCIENCES" TRENDS IN PHARMACOLOGICAL SCIENCES, Bd. 16, 1. September 1995 (1995-09-01), Seiten 309-316, XP002090490 ISSN: 0165-6147**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 071 670 B1

## Beschreibung

Die Erfindung betrifft neue Diphenyl-substituierte 5-Ring-Heterocyclen, Verfahren zu ihrer Herstellung sowie deren Verwendung als Arzneimittel.

Die neuen Verbindungen besitzen die Struktur der allgemeinen Formel (I)

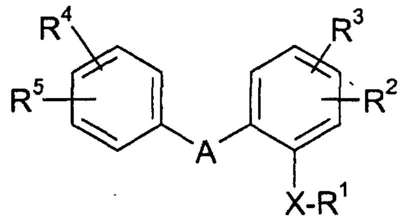

(I)

worin

| | |
|---|---|
| A | ein Furan, Tetrahydrofuran, Dioxolan, Pyrrol, Pyrrolin, Pyrrolidin, Pyrazol, Pyrazolin, Pyrazolidin, Imidazol, Imidazolin, Imidazolidin, Triazol, Triazolin, Triazolidin, Oxazol, Oxazolin, Oxadiazol aber nicht 1,2,4-Oxadiazol, Isoxazol oder Isoxazolin, die jeweils ein- oder mehrfach durch $C_1$-$C_4$-Alkoxy, OH, =O oder $C_1$-$C_4$-Alkyl substituiert ist, wobei der $C_1$-$C_4$-Alkyl-Rest seinerseits substituiert sein kann durch Fluor, Chlor, Brom, OH oder $-NR^6R^7$; |
| X | Sauerstoff; |
| $R^1$ | ein $C_1$-$C_4$-Alkyl-Rest, der durch $-CONHSO_2R^8$, $-CONR^6R^7$, $-CH{=}NOR^8$, $-NR^6R^7$, $-NHCOR^8$, $-NHCONR^6R^7$, $-NHCOOR^8$, $-OCONR^6R^7$, $-SO_2NR^6R^7$ oder durch ein N-Oxid der Formel $-NOR^6R^7$ substituiert ist; |
| $R^2$ und $R^3$ | die gleich oder verschieden sein können Wasserstoff, $-NR^6R^7$, Fluor, Chlor, Brom, Nitro, $CF_3$, CN, $-OR^8$, $C_1$-$C_4$-Alkyl, Phenyl, Benzyl oder Phenyloxy; |
| $R^4$ und $R^5$ | die gleich oder verschieden sein können Wasserstoff, $-NR^6R^7$, Fluor, Chlor, Nitro, $CF_3$, CN, $-OR^8$, $C_1$-$C_4$-Alkyl, Phenyl, Benzyl oder Phenyloxy; |
| $R^6$ | Wasserstoff, $C_3$-$C_6$-Cycloalkyl oder $C_1$-$C_4$-Alkyl, die jeweils ein- oder mehrfach durch Hydroxy, Phenyl, Benzyl oder $C_1$-$C_4$-Alkoxy substituiert sein können, |
| $R^6$ | Phenyl, das gegebenenfalls durch Fluor, Chlor, Brom, $-OR^8$, $C_1$-$C_4$-Alkyl, bevorzugt $-CH_3$, $-SO_3H$, oder $-COOR^8$ substituiert sein kann; |
| $R^7$ | Wasserstoff, $C_3$-$C_6$-Cycloalkyl oder $C_1$-$C_4$-Alkyl, die jeweils ein- oder mehrfach durch Hydroxy, Phenyl, Benzyl oder $C_1$-$C_4$-Alkoxy substituiert sein können, |
| $R^7$ | Phenyl, das gegebenenfalls durch Fluor, Chlor, Brom, $-OR^8$, $C_1$-$C_4$-Alkyl, bevorzugt $-CH_3$, $-SO_3H$, oder $-COOR^8$ substituiert sein kann; oder |
| $R^6$ und $R^7$ | bilden zusammen mit dem Stickstoffatom einen gesättigten oder ungesättigten 5- oder 6-gliedrigen Ring, der als weitere Heteroatome Stickstoff oder Sauerstoff enthalten kann, wobei der Heterocyclus durch $C_1$-$C_4$-Alkyl, Phenyl oder Benzyl substituiert sein kann; |
| $R^8$ | Wasserstoff, $C_1$-$C_4$-Alkyl, ein Benzyl- oder Phenyl-Rest, der gegebenenfalls ein- oder mehrfach durch OH, Chlor, Brom oder $OCH_3$ substituiert ist, bedeuten, gegebenenfalls in Form ihrer Racemate, ihrer Enantiomere, in Form ihrer Diastereomere und ihrer Ge- |

mische, sowie gegebenenfalls ihrer pharmakologisch unbedenklichen Säureadditionssalze.

Bevorzugt sind Verbindungen der allgemeinen Formel (I), worin

A ein Furan, Tetrahydrofuran, Dioxolan, Pyrrol, Pyrrolin, Pyrrolidin, Pyrazol, Pyrazolin, Pyrazolidin, Imidazol, imidazolin, Imidazolidin, Triazol, Triazolin, Triazolidin, Oxazol, Oxazolin, Oxadiazol aber nicht 1,2,4-Oxadiazol, Isoxazol oder Isoxazolin, die jeweils ein- oder mehrfach durch $C_1$-$C_4$-Alkoxy, OH, =O oder $C_1$-$C_4$-Alkyl substituiert sein können, wobei der $C_1$-$C_4$-Alkyl-Rest seinerseits substituiert sein kann durch Fluor, Chlor, Brom, OH oder -$NR^6R^7$;

X Sauerstoff;

$R^1$ $C_1$-$C_4$-Alkyl, das durch -$NR^6R^7$ oder durch ein N-Oxid der Formel -$NOR^6R^7$ substituiert ist;

$R^2$ und $R^3$ die gleich oder verschieden sein können Wasserstoff, -$NR^6R^7$, Fluor, Chlor, Brom, Nitro, $CF_3$, CN, -$OR^8$, $C_1$-$C_4$-Alkyl, Phenyl, Benzyl oder Phenyloxy;

$R^4$ und $R^5$ die gleich oder verschieden sein können Wasserstoff, -$NR^6R^7$, Fluor, Chlor, Nitro, $CF_3$, CN, -$OR^8$, $C_1$-$C_4$-Alkyl, Phenyl, Benzyl oder Phenyloxy;

$R^6$ Wasserstoff oder $C_1$-$C_4$-Alkyl, das ein- oder mehrfach durch Hydroxy, Phenyl, Benzyl oder $C_1$-$C_4$-Alkoxy substituiert sein kann,

$R^7$ Wasserstoff oder $C_1$-$C_4$-Alkyl, das ein- oder mehrfach durch Hydroxy, Phenyl, Benzyl oder $C_1$-$C_4$-Alkoxy substituiert sein kann, oder

$R^6$ und $R^7$ bilden zusammen mit dem Stickstoffatom einen gesättigten oder ungesättigten Heterocyclus ausgewählt aus der Gruppe Pyrrol, Pyrrolin, Pyrrolidin, Piperidin, Piperazin, Morpholin, Thiomorpholin, Imidazol, Imidazolin, Imidazolidin, Pyrazol, Pyrazolin, Pyrazolidin, wobei die genannten Heterocyclen gegebenenfalls durch Methyl, Ethyl, Propyl oder Benzyl substituiert sein können;

$R^8$ Wasserstoff, $C_1$-$C_4$-Alkyl, Benzyl oder Phenyl bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Besonders bevorzugt sind Verbindungen der allgemeinen Formel (I), worin

A ein Rest ausgewählt aus der Gruppe Pyrrol, Pyrazol, Imidazol, Imidazolin, Imidazolidin, Triazol, Oxazol, Oxazolin, Isoxazol, 1,3,4-Oxadiazol oder Oxadiazolin, der gegebenenfalls ein- oder mehrfach durch OH, =O, $C_1$-$C_4$-Alkyloxy oder durch $C_1$-$C_4$-Alkyl substituiert sein kann, wobei der $C_1$-$C_4$-Alkyl-Rest seinerseits substituiert sein kann durch Chlor, Brom, OH oder -$NR^6R^7$;

X Sauerstoff;

$R^1$ $C_1$-$C_4$-Alkyl, das durch -$NR^6R^7$ substituiert ist;

$R^2$ und $R^3$ Wasserstoff;

$R^4$ und $R^5$ Wasserstoff;

$R^6$ Wasserstoff, Methyl, Ethyl, Propyl oder Butyl;

$R^7$ Wasserstoff, Methyl, Ethyl, Propyl oder Butyl; oder

$R^6$ und $R^7$ bilden zusammen mit dem Stickstoffatom einen gesättigten oder ungesättigten Heterocyclus ausgewählt aus der Gruppe Piperidin, Piperazin oder Morpholin, die gegebenenfalls durch Methyl, Ethyl oder Benzyl substituiert sein können, bedeuten, gegebenenfalls in Form ihrer Racemate, ihrer Enantiomere, in Form ihrer Diastereomere und ihrer Gemische, sowie gegebenenfalls ihrer pharmakologisch unbedenklichen

Säureadditionssalze.

Erfindungsgemäß bevorzugt sind ferner Verbindungen der allgemeinen Formel (I), worin

A ein Rest ausgewählt aus der Gruppe Pyrrol, Pyrazol, Imidazol, Imidazolin, Imidazolidin, Triazol, Oxazol, Oxazolin, Isoxazol, 1,3,4-Oxadiazol oder Oxadiazolin, der gegebenenfalls ein- oder mehrfach durch OH, =O, $C_1$-$C_4$-Alkyloxy oder durch $C_1$-$C_4$-Alkyl substituiert sein kann, wobei der $C_1$-$C_4$-Alkyl-Rest seinerseits substituiert sein kann durch Chlor, Brom, OH oder $-NR^6R^7$;

X Sauerstoff;

$R^1$ ein Ethyl- oder Propyl-Rest, der durch $-NR^6R^7$ substituiert ist;

$R^2$ und $R^3$ Wasserstoff;

$R^4$ und $R^5$ Wasserstoff;

$R^6$ Wasserstoff, Methyl, Ethyl, Propyl oder Butyl;

$R^7$ Wasserstoff, Methyl, Ethyl, Propyl oder Butyl, bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

Erfindungsgemäß besonders bevorzugt sind Verbindungen der allgemeinen Formel (I), worin die Gruppe

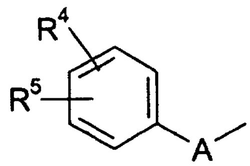

für einen der folgenden Reste stehen kann

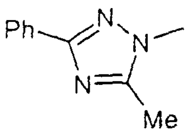 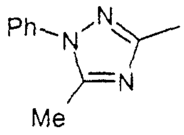 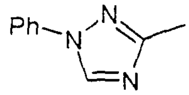 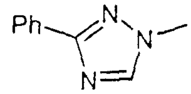

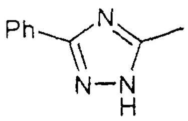 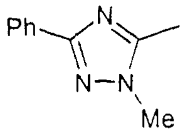 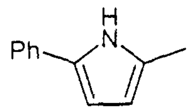 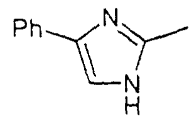

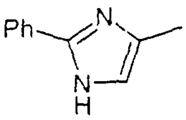 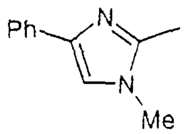 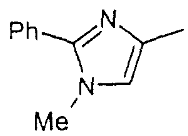 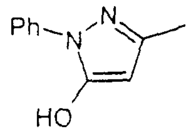

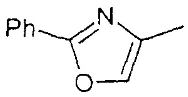 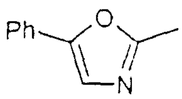 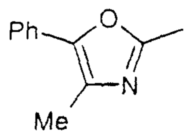 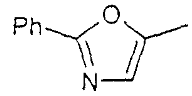

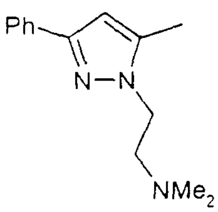 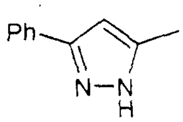 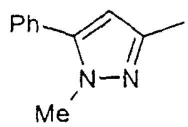 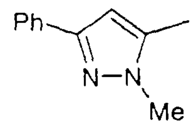

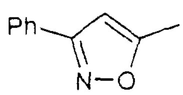 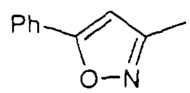 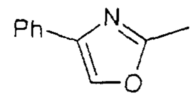

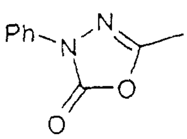 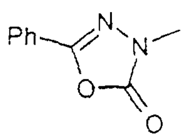 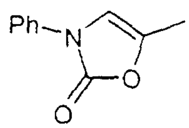 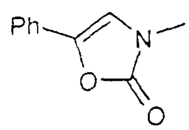

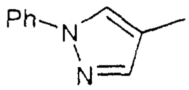 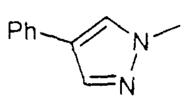 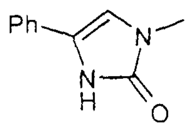 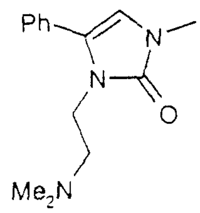

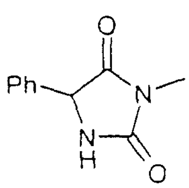 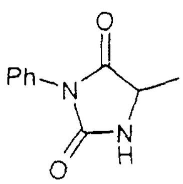 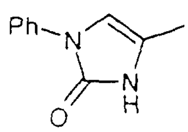

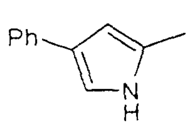 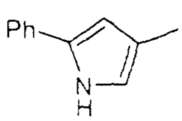 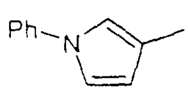 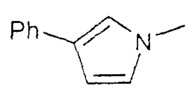

:

X Sauerstoff;

$R^1$ ein Ethyl- oder Propyl-Rest, der durch $-NR^6R^7$ substituiert ist;

$R^2$ und $R^3$ Wasserstoff;

$R^4$ und $R^5$ Wasserstoff;

$R^6$ Wasserstoff, Methyl, Ethyl, Propyl oder Butyl;

$R^7$ Wasserstoff, Methyl, Ethyl, Propyl oder Butyl, bedeuten, gegebenenfalls in Form ihrer Racemate, ihrer Enantiomere, in Form ihrer Diastereomere und ihrer Gemische, gegebenenfalls in Form ihrer Tautomere, sowie gegebenenfalls ihrer pharmakologisch unbedenklichen Säureadditionssalze.

Ferner sind erfindungsgemäß besonders bevorzugt Verbindungen der allgemeinen Formel (I), worin die Gruppe

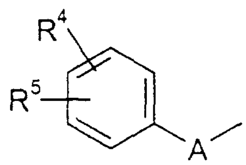

für einen der folgenden Reste stehen kann:

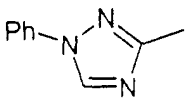
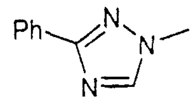
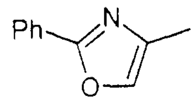
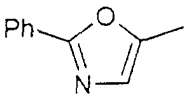
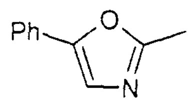
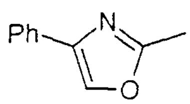
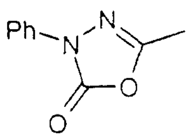
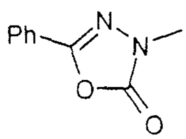
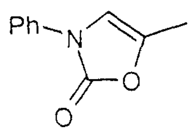
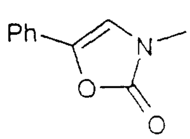
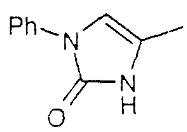
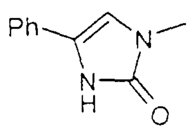

X Sauerstoff;

$R^1$ ein Ethyl- oder Propyl-Rest, der durch $-NR^6R^7$ substituiert ist;

$R^2$ und $R^3$ Wasserstoff;

$R^4$ und $R^5$ Wasserstoff;

$R^6$ Wasserstoff, Methyl, Ethyl, Propyl oder Butyl;

$R^7$ Wasserstoff, Methyl, Ethyl, Propyl oder Butyl, bedeuten, gegebenenfalls in Form ihrer Racemate, ihrer Enantiomere, in Form ihrer Diastereomere und ihrer Gemische, gegebenenfalls in Form ihrer Tautomere,

sowie gegebenenfalls ihrer pharmakologisch unbedenklichen Säureadditionssalze.

Ferner sind von besonderer Bedeutung Verbindungen der allgemeinen Formel (I), worin die Gruppe

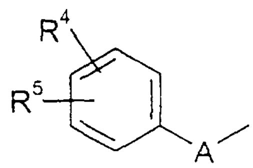

für einen der folgenden Reste stehen kann:

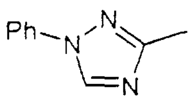

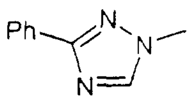

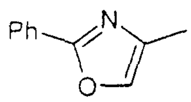

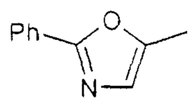

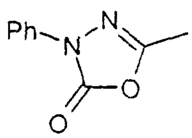

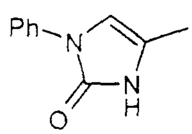

;

| | |
|---|---|
| X | Sauerstoff; |
| $R^1$ | $-CH_2-CH_2-NR^6R^7$ ; |
| $R^2$ und $R^3$ | Wasserstoff; |
| $R^4$ und $R^5$ | Wasserstoff; |
| $R^6$ | Methyl; |
| $R^7$ | Methyl, bedeuten, gegebenenfalls in Form ihrer Racemate, ihrer Enantiomere, in Form ihrer Diastereomere und ihrer Gemische, gegebenenfalls in Form ihrer Tautomere, sowie gegebenenfalls ihrer pharmakologisch unbedenklichen Säureadditionssalze. |

Als besonders bevorzugt seien ferner die folgenden Verbindungen hervorgehoben:

-3-{2-[2(N,N-Dimethylamino)ethyl]oxy-phenyl}-1-phenyl-1,2,4-triazol;
-1-{2-[2(N,N-Dimethylamino)ethyl]oxy-phenyl}-3-phenyl-1,2,4-triazol;
-4-{2-[2(N,N-Dimethylamino)ethyl]oxy-phenyl}-2-phenyl-1,3-oxazol;
-5-{2-[2(N,N-Dimethylamino)ethyl]oxy-phenyl}-2-phenyl-1,3-oxazol;

Als Alkylgruppen (auch soweit sie Bestandteil anderer Reste sind, beispielsweise Alkylenbrücken) werden, soweit nicht anders angegeben, verzweigte und unverzweigte Alkylgruppen mit 1 bis 10 Kohlenstoffatomen bevorzugt 1 - 4 Kohlenstoffatomen bezeichnet, beispielsweise werden genannt: Methyl, Ethyl, n-Propyl, iso-Propyl, Butyl, iso-Butyl, sec. Butyl, tert.-Butyl, Pentyl, iso-Pentyl, Hexyl, Heptyl und Octyl. Zur Bezeichnung der Gruppen Methyl, Ethyl, Butyl oder auch tert.-Butyl werden auch die Abkürzungen Me, Et, Bu oder tBu verwendet.

Substituierte Alkylgruppen können, sofern nicht anders beschrieben (auch soweit sie Bestandteil anderer Reste sind), beispielsweise einen oder mehrere der nachfolgend genannten Substituenten tragen: Halogen, Hydroxy, Mercapto, $C_1$-$C_6$-Alkyloxy, Amino, Alkylamino, Dialkylamino, Cyano, Nitro, =O, -CHO, -COOH, $-COO-C_1-C_6$-Alkyl, $-S-C_1-C_6$-Alkyl.

Als Alkenylgruppen (auch soweit sie Bestandteil anderer Reste sind) werden verzweigte und unverzweigte Alkenylgruppen mit 2 bis 10 Kohlenstoffatomen, bevorzugt 2 bis 3 Kohlenstoffatomen genannt, soweit sie mindestens

eine Doppelbindung aufweisen, beispielsweise auch oben genannte Alkylgruppen bezeichnet soweit sie mindestens eine Doppelbindung aufweisen, wie zum Beispiel Vinyl (soweit keine unbeständigen Enamine oder Enolether gebildet werden), Propenyl, iso-Propenyl, Butenyl, Pentenyl, Hexenyl.

Substituierte Alkenylgruppen können, sofern nicht anders beschrieben (auch soweit sie Bestandteil anderer Reste sind), beispielsweise einen oder mehrere der nachfolgend genannten Substituenten tragen: Halogen, Hydroxy, Mercapto, $C_1$-$C_6$-Alkyloxy, Amino, Alkylamino, Dialkylamino, Cyano, Nitro, =O, -CHO, -COOH, -COO-$C_1$-$C_6$-Alkyl, -S-$C_1$-$C_6$-Alkyl.

Als Alkinylgruppen (auch soweit sie Bestandteil anderer Reste sind) werden Alkinylgruppen mit 2 bis 10 Kohlenstoffatomen bezeichnet, soweit sie mindestens eine Dreifachbindung aufweisen, beispielsweise Ethinyl, Propargyl, Butinyl, Pentinyl, Hexinyl.

Substituierte Alkinylgruppen können, sofern nicht anders beschrieben (auch soweit sie Bestandteil anderer Reste sind), beispielsweise einen oder mehrere der nachfolgend genannten Substituenten tragen: Halogen, Hydroxy, Mercapto, $C_1$-$C_6$-Alkyloxy, Amino, Alkylamino, Dialkylamino, Cyano, Nitro, =O, -CHO, -COOH, -COO-$C_1$-$C_6$-Alkyl, -S-$C_1$-$C_6$-Alkyl.

Als Cycloalkylreste mit 3 - 6 Kohlenstoffatomen werden beispielsweise Cyclopropyl, Cyclobutyl, Cyclopentyl oder Cyclohexyl bezeichnet, die auch durch verzweigtes oder unverzweigtes Alkyl mit 1 bis 4 Kohlenstoffatomen, Hydroxy, und/oder Halogen oder wie zuvor definiert substituiert sein können. Als Halogen wird im allgemeinen Fluor, Chlor, Brom oder Jod bezeichnet.

Der Begriff Aryl steht für ein aromatisches Ringsystem mit 6 bis 10 Kohlenstoffatomen, das, soweit nicht anders beschrieben, beispielsweise einen oder mehrere der nachfolgend genannten Substituenten tragen kann: $C_1$-$C_6$-Alkyl, $C_1$-$C_6$-Alkyloxy, Halogen, Hydroxy, Mercapto, Amino, Alkylamino, Dialkylamino, $CF_3$, Cyano, Nitro, -CHO, -COOH, -COO-$C_1$-$C_6$-Alkyl, -S-$C_1$-$C_6$-Alkyl. Bevorzugter Arylrest ist Phenyl.

Als Beispiele für N-verknüpfte cyclische Reste der allgemeinen Formel $NR^6R^7$ seien genannt: Pyrrol, Pyrrolin, Pyrrolidin, 2-Methylpyrrolidin, 3-Methylpyrrolidin, Piperidin, Piperazin, N-Methylpiperazin, N-Ethylpiperazin, N-(n-Propyl)-piperazin, N-Benzylpiperazin, Morpholin, Thiomorpholin, Imidazol, Imidazolin, Imidazolidin, Pyrazol, Pyrazolin, Pyrazolidin, bevorzugt Morpholin, N-Benzylpiperazin, Piperazin, und Piperidin, wobei die genannten Heterocyclen durch Alkyl mit 1 bis 4 Kohlenstoffatomen, bevorzugt Methyl substituiert sein können.

"=O" bedeutet ein über eine Doppelbindung verknüpftes Sauerstoffatom.

Die vorliegende Erfindung beschreibt Verbindungen, die überraschenderweise eine hohe Affinität zu folgendem Rezeptortyp aufweist: "Na+ Kanal site 2" Bindungsstelle. Darüber hinaus zeigen diese Verbindungen antagonistische Aktivität am AMPA-Rezeptor. Aufgrund dieser Befunde können die erfindungsgemäßen Verbindungen bei neurodegenerativen Erkrankungen sowie Gehirnischämie verschiedener Genese eingesetzt werden.

Die erfindungsgemäßen Verbindungen können in Anlehnung an bekannte Analogieverfahren dargestellt werden. So können beispielsweise Verbindungen der allgemeinen Formel (II)

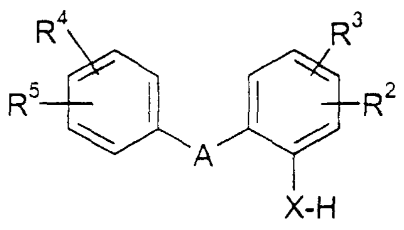

(II),

worin A, X und die Reste $R^2$, $R^3$, $R^4$ und $R^5$ die oben genannte Bedeutung aufweisen, unter basischen Bedingungen mit Elektrophilen der allgemeinen Formel

$$L\text{-}R^1,$$

wobei L eine Abgangsgruppe wie beispielsweise Chlor, Brom, Iod, Methansulfonyl, Trifluormethansulfonyl oder p-Toluolsulfonyl darstellt und $R^1$ die oben angegebene Bedeutung aufweist, unter Alkylierung zu den Verbindungen der allgemeinen Formel (I) umgesetzt werden.

Die folgenden Synthesevorschriften dienen der näheren Erläuterung der zur Darstellung der erfindungsgemäßen Verbindungen anwendbaren Analogieverfahren, ohne die Erfindung jedoch auf deren Gegenstand zu beschränken.

**Beispiel 1:** 1,3-Diphenyl-1,2,4-triazol

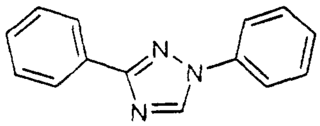

a) Benzoylimidoethylester-hydrochlorid:

Bei ca. 0°C wird in eine Lösung von 100 g Benzonitril und 60 ml Ethanol über einen Zeitraum von etwa 3 Stunden HCl-Gas eingeleitet (Gewichtszunahme: ca. 65 g). Die erhaltene Mischung wird anschließend 6 Tage bei -25°C stehen gelassen. Nach Erwärmen auf 0°C wird mit Ether verrührt und der so erhaltene kristalline Niederschlag abgesaugt, mit Ether nachgewaschen und getrocknet. Das so erhaltene Imidoetherhydrochlorid (139,5 g = 78% der Th.) wird ohne weitere Reinigung in die nächste Reaktionsstufe eingesetzt.

b) Imidohydrazid:

In 50 ml wasserfreiem Ethanol werden 3,7 g Benzoylimidoethylester-hydrochlorid und 2,2 g Phenylhydrazin über einen Zeitraum von 1,5 Stunden zum Rückfluß erhitzt. Anschließend wird das Lösemittel im Vakuum abdestilliert und der verbleibende Rückstand in Wasser aufgenommen. Nach Extraktion mit Diethylether wird die wässrige Phase mit wässriger Ammoniak-Lösung alkalisch gestellt und erneut mit Diethylether extrahiert. Die vereinigten organischen Phasen werden über $Na_2SO_4$ getrocknet. Nach Abdestillieren des Lösemittels im Vakuum verbleiben 2,3 g (54%) des Imidohydrazids in Form leicht roter Kristalle. Eine weitergehende Reinigung erfolgt nicht.

c) 1,3-Diphenyl-1,2,4-triazol

2 g Imidohydrazid werden in 5 ml Ameisensäure (98%-ig) aufgenommen und 2 Stunden zum Rückfluß erhitzt. Nach Abkühlen auf Raumtemperatur wird der Ansatz vorsichtig mit 10%iger $Na_2CO_3$-Lösung auf pH=8 gestellt und die erhaltene Mischung zweimal mit Ethylacetat extrahiert. Die vereinigten organischen Phasen werden über $MgSO_4$ getrocknet und das Lösemittel im Vakuum abdestilliert. Der verbleibende Rückstand wird durch Chromatographie an Kieselgel (Dichlormethan:Methanol 95:5) gereinigt und anschließend ins Hydrochlorid überführt. Ausbeute: 0,7 g (30%), Schmp.: 80°C;

**Beispiel 2:** 1-(2-Methoxyphenyl)-3-phenyl-1,2,4-triazol

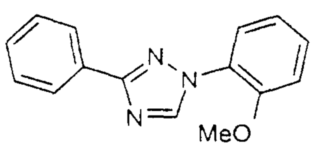

a) Imidohydrazidbildung:

In 80 ml wasserfreiem Ethanol werden 4,6 g Benzoylimidoethylester-hydrochlorid (siehe Beispiel 1, Stufe a) und 3,5 g 2-Methoxy-phenylhydrazin über einen Zeitraum von 4-5 Stunden zum Rückfluß erhitzt. Anschließend wird das Lösemittel im Vakuum abdestilliert und der verbleibende Rückstand in verdünnter wässriger HCl-Lösung aufgenommen. Nach Extraktion mit Diethylether wird die wässrige Phase mit wässriger Ammoniak-Lösung alkalisch gestellt und erneut mit Diethylether extrahiert. Die vereinigten organischen Phasen werden über $Na_2SO_4$ getrocknet. Nach Abdestillieren des Lösemittels im Vakuum verbleiben 2,5 g (41%) des Imidohydrazids in Form eines braunen Öls. Eine weitergehende Reinigung erfolgt nicht.

b) 1-(2-Methoxyphenyl)-3-phenyl-1 2,4-triazol

2,4 g imidohydrazid werden in 10 ml Ameisensäure (98%-ig) aufgenommen und 8-9 Stunden zum Rückfluß erhitzt. Nach Abkühlen auf Raumtemperatur wird der Ansatz auf Eis gegeben und die erhaltene Mischung zweimal mit Ethylacetat extrahiert. Die vereinigten organischen Phasen werden über $MgSO_4$ getrocknet und das Lösemittel im Vakuum abdestilliert. Die Reinigung des verbleibenden Rückstands erfolgt durch Chromatographie an Kieselgel (Toluol: Ethylacetat 90:10). Ausbeute: 1,2 g (48%), Schmp.: 83-84°C;

**Beispiel 3:** 1-{2-[2(N,N-Dimethylamino)ethyl]oxy-phenyl}-3-phenyl-1,2,4-triazol

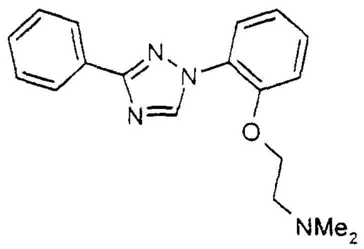

a) 1-(2-Hydroxyphenyl)-3-phenyl-1,2,4-triazol

1,1 g 1-(2-Methoxyphenyl)-3-phenyl-1,2,4-triazol (siehe Beispiel 2) werden in 40 ml Dichlormethan gelöst und tropfenweise mit einer Lösung von 1 ml $BBr_3$ in 10 ml Dichlormethan versetzt. Nach 3-4 Stunden Rühren bei Raumtemperatur wird der Ansatz auf Eiswasser gegeben und die organische Phase abgetrennt. Die verbleibende wässrige Phase wird nochmals mit Dichlormethan extrahiert. Nach Trocknen der gesammelten Dichlormethanphasen über $Na_2SO_4$ wird das Lösemittel abdestilliert. Es verbleiben 1 g (96%) der Titelverbindung (Schmp.: 148-154°C).

b) 1-{2-[2(N,N-Dimethylamino)ethyl]oxy-phenyl}-3-phenyl-1,2,4-triazol

0,94 g 1-(2-Hydroxyphenyl)-3-phenyl-1,2,4-triazol werden in 25 ml DMF gelöst und mit 0,18 g Natriumhydrid (60% in Öl) versetzt. Man rührt anschließend noch 30 Minuten bei Raumtemperatur und gibt dann eine zuvor 5 Minuten gerührte Mischung aus 1,15 g 2-N,N-Dimethylaminoethylchlorid und 0,36 g Natriumhydrid (60% in Öl) in 25 ml DMF zu. Diese Mischung wird für 4 Stunden auf 100°C erhitzt, und anschließend im Vakuum vom Lösemittel befreit. Der Rückstand wird in 2 N Salzsäure aufgenommen und mit Essigsäureethylester extrahiert. Die wässrige Phase wird mit wässriger Ammoniaklösung alkalisch gestellt und zweimal mit Dichlormethan extrahiert. Die organische Phase wird über Natriumsulfat getrocknet, im Vakuum eingeengt und an Kieselgel chromatographiert (Methanol).
Ausbeute: 500 mg (41%), helles Öl; Schmp. (Fumarat): 184-186°C;

**Beispiel 4:** 1-{2-[2(N,N-Dimethylamino)ethyl]oxy-phenyl}-5-methyl-3-phenyl-1,2,4-triazol

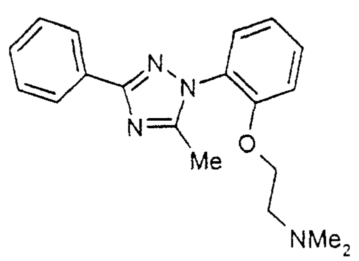

a) Imidohydrazidbildung:

In 40 ml wasserfreiem Ethanol werden 2,5 g des gemäß Beispiel 1 (Stufe a) erhaltenen Benzoylimidoethylester-hydrochlorids und 2,9 g 2-Benzyloxyphenylhydrazin über einen Zeitraum von 2 Stunden zum Rückfluß erhitzt. Anschließend wird das Lösemittel im Vakuum abdestilliert und der verbleibende Rückstand in verdünnter wässriger HCl-Lösung aufgenommen. Nach Extraktion mit Diethylether wird die wässrige Phase mit wässriger Ammoniak-Lösung alkalisch gestellt und erneut mit Diethylether extrahiert. Die vereinigten organischen Phasen werden über $Na_2SO_4$ getrocknet. Nach Abdestillieren des Lösemittels im Vakuum verbleiben 1,6 g (37%) des Imidohydrazids in Form leicht brauner Kristalle. Eine weitergehende Reinigung erfolgt nicht.

b) 1-(2-Benzyloxyphenyl)-5-methyl-3-phenyl-1,2,4-triazol

1,55 g Imidohydrazid werden mit 4,1 g Orthoessigsäuretriethylester in 30 ml wasserfreiem Ethanol aufgenommen und 6 Stunden zum Rückfluß erhitzt. Nach Abkühlen auf Raumtemperatur wird das Lösemittel im Vakuum abdestilliert und der verbleibende Rückstand in Diethylether aufgenommen. Die so erhaltene organische Phase wird dreimal mit Wasser gewaschen, über $MgSO_4$ getrocknet und das Lösemittel im Vakuum abdestilliert. Die Reinigung des verbleibenden Rohprodukts erfolgt durch Chromatographie an Kieselgel (Toluol: Ethylacetat 80:20).
Ausbeute: 1,1 g (64%), leicht bräunliches Öl;

c) 1-(2-Hydroxyphenyl)-5-methyl-3-phenyl-1,2,4-triazol

1 g 1-(2-Benzyloxyphenyl)-5-methyl-3-phenyl-1,2,4-triazol werden in 20 ml wasserfreiem Dichlormethan gelöst und tropfenweise mit einer Lösung von 0,5 ml $BBr_3$ in 10 ml Dichlormethan versetzt. Nach 3 Stunden Rühren bei Raumtemperatur wird der Ansatz mit weiteren 100 ml Dichlormethan verdünnt und zweimal mit Wasser gewaschen. Nach Trocknen der organischen Phase über $Na_2SO_4$ wird das Lösemittel abdestilliert. Die Reinigung des verbleibenden Rohprodukts erfolgt durch Chromatographie an Kieselgel (Toluol:Ethylacetat 80:20).
Ausbeute: 0,6 g (82%); Schmp.: 198-199°C, farblose Kristalle;

d) 1-{2-[2(N,N-Dimethylamino)ethyl]oxy-phenyl}-5-methyl-3-phenyl-1,2,4-triazol

0,69 g 2-N,N-Dimethylaminoethylchlorid x HCl werden in 10 ml 1,4-Dioxan und 2 ml Wasser vorgelegt, mit 0,69 g Kaliumcarbonat versetzt und 30 Minuten bei Raumtemperatur gerührt. Anschließend erfolgt die langsame Zugabe einer Lösung von 0,6 g 1-(2-Hydroxyphenyl)-5-methyl-3-phenyl-1,2,4-triazol in 5 ml 1,4-Dioxan sowie einer Suspension von 0,27 g Kalium-tert.-butylat in 5 ml 1,4-Dioxan. Die so erhaltene Mischung wird 3 Stunden bei ca. 100°C gerührt. Anschließend wird das Lösemittel im Vakuum abdestilliert, der verbleibende Rückstand in Ethylacetat aufgenommen und die so erhaltene organische Phase erst mit Wasser, dann mit 2N-HCl-Lösung (aq.) extrahiert. Die wässrigen Phasen werden mit wässriger Ammoniaklösung alkalisch gestellt und mit Ethylacetat extrahiert. Nach Trocknen der vereinigten organischen Phasen über $MgSO_4$ wird das Lösemittel im Vakuum abdestilliert. Die Reinigung des verbleibenden Rohprodukts erfolgt durch Chromatographie an Kieselgel (Dichlormethan;Methanol 80:20). Die so erhaltene Titelverbindung wird in das Dihydrochlorid überführt.
Ausbeute: 0,4 g (42%); Schmp.: 170°C (Zersetzung), farblose Kristalle;

**Beispiel 5:** 3-{2-[2(N,N-Dimethylamino)ethyl]oxy-phenyl}-1-phenyl-1,2,4-triazol

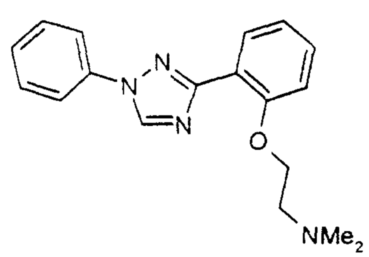

a) 2-Hydroxy-benzoylimidoethylester-hydrochlorid:

Bei ca. 0°C wird in eine Lösung von 60 g 2-Cyanophenol und 60 ml Ethanol in 500 ml wasserfreiem Diethylether über einen Zeitraum von etwa 3 Stunden HCl-Gas eingeleitet. Die erhaltene Mischung wird 2 Stunden bei gleichbleibender Temperatur nachgerührt und anschließend mehrere Tage stehen gelassen. Der kristalline Niederschlag wird abgesaugt, mit Ether nachgewaschen und getrocknet. Das so erhaltene Imidoetherhydrochlorid (49,1 g = 48%; Schmp.: 130-131°C) wird ohne weitere Reinigung in die nächste Reaktionsstufe eingesetzt.

b) Imidohydrazidbildung:

In 50 ml wasserfreiem Ethanol werden 4 g des zuvor hergestellten 2-Hydroxybenzoylimidoethylester-hydrochlorids und 2,2 g Phenylhydrazin über einen Zeitraum von 2 Stunden zum Rückfluß erhitzt. Anschließend wird das Lösemittel im Vakuum abdestilliert und der verbleibende Rückstand in verdünnter wässriger HCl-Lösung aufgenommen. Nach Extraktion mit Diethylether wird die wässrige Phase mit wässriger Ammoniak-Lösung alkalisch gestellt und erneut mit Diethylether extrahiert. Die vereinigten organischen Phasen werden über $Na_2SO_4$ getrocknet. Nach Abdestillieren des Lösemittels im Vakuum verbleiben 3,4 g (75%) des Imidohydrazids in Form leicht roter Kristalle (Schmp.: 154°C u. Zersetzung). Eine weitergehende Reinigung erfolgt nicht.

c) 3-(2-Hydroxyphenyl)-1-phenyl-1,2,4-triazol

3 g Imidohydrazid werden in 7,5 ml Ameisensäure (98%-ig) aufgenommen und 10 Stunden zum Rückfluß erhitzt. Nach Abkühlen auf Raumtemperatur wird der Ansatz auf Eis gegeben und vorsichtig mit 10%-iger $Na_2CO_3$-Lösung (aq.) neutralisiert. Die erhaltene Mischung wird zweimal mit Ethylacetat extrahiert. Die vereinigten organischen Phasen werden über $Na_2SO_4$ getrocknet und das Lösemittel im Vakuum abdestilliert. Die Reinigung des verbleibenden Rückstands erfolgt durch Chromatographie an Kieselgel (Toluol:Ethylacetat 80:20).
Ausbeute: 2,6 g (84%), Schmp.: 105°C;

d) 3-{2-[2(N,N-Dimethylamino)ethyl]oxy-phenyl}-1-phenyl-1,2,4-triazol

2,37 g 3-(2-Hydroxyphenyl)-1-phenyl-1,2,4-triazol werden in 50 ml DMF gelöst und mit 0,44 g Natriumhydrid (60% in Öl) versetzt. Man rührt anschließend noch 30 Minuten bei Raumtemperatur nach und gibt dann eine zuvor 5 Minuten gerührte Mischung aus 2,88 g 2-N,N-Dimethylaminoethylchlorid und 0,88 g Natriumhydrid (60% in Öl) in 50 ml DMF zu. Diese Mischung wird für 4 Stunden auf 100°C erhitzt, und anschließend im Vakuum vom Lösemittel befreit. Der Rückstand wird in 2 N HCl-Lösung (aq.) aufgenommen und mit Essigsäureesthylester extrahiert. Die wässrige Phase wird mit wässriger Ammoniaklösung alkalisch gestellt und mit Dichlormethan extrahiert. Die organische Phase wird über Natriumsulfat getrocknet, im Vakuum eingeengt und an Kieselgel chromatographiert (Methanol). Nach der Reinigung wird die Titelverbindung in das Hydrochlorid überführt.
Ausbeute: 2,5 g (72%), Schmp. (Hydrochlorid): 187-198°C;

**Beispiel 6:** 3-{2-[2(N,N-Dimethylamino)ethyl]oxy-phenyl}-5-methyl-1-phenyl-1,2,4-triazol

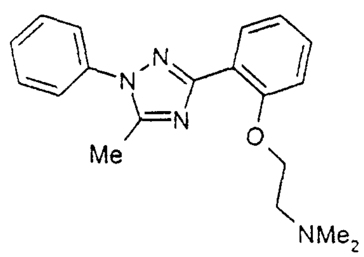

a) 3-(2-Hydroxyphenyl)-5-methyl-1-phenyl-1,2,4-triazol

3,4 g des gemäß Beispiel 5 (Stufe b) hergestellten Imidohydrazids werden mit 12,1 g Orthoessigsäuretriethylester in 50 ml wasserfreiem Ethanol aufgenommen und 6 Stunden zum Rückfluß erhitzt. Nach Abkühlen auf Raumtemperatur wird das Lösemittel im Vakuum abdestilliert und der verbleibende Rückstand in Diethylether aufgenommen.

Die so erhaltene organische Phase wird dreimal mit Wasser gewaschen, über $MgSO_4$ getrocknet und das Lösemittel im Vakuum abdestilliert. Die Reinigung des verbleibenden Rohprodukts erfolgt durch Chromatographie an Kieselgel (Toluol:Ethylacetat 80:20).
Ausbeute: 2 g (53%), leicht bräunliches Öl;

b) 3-{2-[2(N,N-Dimethylamino)ethyl]oxy-phenyl}-5-methyl-1-phenyl-1,2,4-triazol

2,3 g 2-N,N-Dimethylaminoethylchlorid x HCl werden in 10 ml 1,4-Dioxan und 2 ml Wasser vorgelegt, mit 2,8 g Kaliumcarbonat versetzt und 30 Minuten bei Raumtemperatur gerührt. Anschließend erfolgt die langsame Zugabe einer Lösung von 2,0 g 3-(2-Hydroxyphenyl)-5-methyl-1-phenyl-1,2,4-triazol in 10 ml 1,4-Dioxan sowie einer Suspension von 0,9 g Kalium-tert.-butylat in 5 ml 1,4-Dioxan. Die so erhaltene Mischung wird 3 Stunden bei ca. 100°C gerührt. Anschließend wird das Lösemittel im Vakuum abdestilliert, der verbleibende Rückstand in Ethylacetat aufgenommen und die so erhaltene organische Phase erst mit Wasser, dann mit 2N-HCl-Lösung (aq.) extrahiert. Die wässrigen Phasen werden mit wässriger Ammoniaklösung alkalisch gestellt und mit Ethylacetat extrahiert. Nach Trocknen der vereinigten organischen Phasen über $MgSO_4$ wird das Lösemittel im Vakuum abdestilliert. Die Reinigung des verbleibenden Rohprodukts erfolgt durch Chromatographie an Kieselgel (Dichlormethan:Methanol 80:20). Die so erhaltene Titelverbindung wird in das Dihydrochlorid überführt.
Ausbeute: 1,2 g (38%); Schmp.: 85°C (Zersetzung), farblose Kristalle;

**Beispiel 7:** 3-{2-[2(N,N-Dimethylamino)ethyl]oxy-phenyl}-5-phenyl-1,2,4-triazol

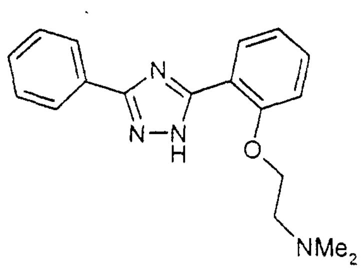

a) 2-Phenyl-benzo[e][1,3]oxazin-4-on:

Die Herstellung erfolgte nach einem literaturbekannten Verfahren (J. Chem. Soc. 1910,208).

b) 3-(2-Hydroxyoxyphenyl)-5-phenyl-1,2,4-triazol:

1,9 g 2-Phenyl-benzo[e][1,3]oxazin-4-on werden in 75 ml Ethanol mit 1,0 g Hydrazinhydrat zum Rückfluß erhitzt. Nach ca. 1 Stunde wird das Lösemittel im Vakuum abdestilliert und der verbleibende Rückstand in Wasser verrührt. Der entstehende Feststoff (3-(2-Hydroxyphenyl)-5-phenyl-1,2,4-triazol) wird ohne weitere Reinigung in die nächste Stufe eingesetzt. Ausbeute: 0,5 g (25%);

c) 3-{2-(2N,N-Dimethylamino)ethyl]oxy-phenyl}-5-phenyl-1,2,4-triazol

0,85 g 3-(2-Hydroxyphenyl)-5-phenyl-1,2,4-triazol werden mit 4 mmol Natriummethylat bei Raumtemperatur für 15 Minuten in wasserfreiem Methanol (20 ml) gerührt. Nach Abdestillieren des Lösemitels im Vakuum werden 20 ml DMF zu gegeben und die so erhaltene Lösung zu einer zuvor 30 Minuten gerührten Mischung aus 0,87 g 2-N, N-Dimethylaminoethylchlorid und 0,24 g Natriumhydrid (60% in Öl) in 20 ml DMF gegeben. Diese Mischung wird für 2 Stunden auf 100°C erhitzt, und anschließend im Vakuum vom Lösemittel befreit. Der Rückstand wird in Wasser aufgenommen und zweimal mit Dichlormethan extrahiert. Die organische Phase wird über Natriumsulfat getrocknet, im Vakuum eingeengt und an Kieselgel chromatographiert (Ethylacetat:Isopropanol 70:30). Nach der Reinigung wird die Titelverbindung in das Hydrochlorid überführt.
Ausbeute: 0,14 g (11%), Schmp. (Hydrochlorid): 207-208°C;

**Beispiel 8:** 4-{2-[2(N,N-Dimethylamino)ethyl]oxy-phenyl}-2-phenyl-1,3-oxazol

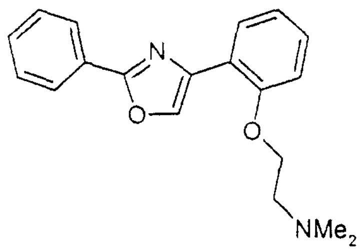

a) 2-Benzyloxyacetophenon:

2,3 g Natrium werden in 150 ml wasserfreiem Methanol gelöst und mit 13,6 g 2-Hydroxyacetophenon versetzt. Nach 10 Minuten Rühren bei Raumtemperatur werden 17,1 g Benzylbromid und 10 ml DMF zugesetzt. Es wird ca. 4 Stunden zum Rückfluß erhitzt. Zur Aufarbeitung wird das Lösemittel im Vakuum abdestilliert, der Rückstand in Ethylacetat aufgenommen und mit Wasser gewaschen. Die wässrige Phase wird nochmals mit Ethylacetat extrahiert. Nach Trocknen der vereinigten organischen Phasen über $Na_2SO_4$ wird das Lösemittel im Vakuum abdestilliert. Es verbleiben 19 g (84%) 2-Benzyloxyacetophenon, die ohne weitere Reinigung in die nächste Stufe eingesetzt werden können.

b) 1-(2-Benzyloxy-phenyl)-2-hydroxy-ethanon:

2,26 g des 2-Benzyloxyacetophenons aus Stufe (a) werden mit 3,54 g Diacetoxyphenyl-$\lambda^3$-iodan in 22 ml Methanol aufgenommen und bei ca. 10°C unter Rühren langsam mit einer Lösung von 1,2 g NaOH in 22 ml Methanol versetzt. Nach einer Stunde Rühren bei Raumtemperatur wird der Ansatz unter Kühlung mit 2N HCl-Lösung (aq.) angsäuert und zweimal mit Dichlormethan extrahiert Nach Trocknen der vereinigten organischen Phasen über $Na_2SO_4$ wird das Lösemittel im Vakuum abdestilliert und der verbleibende Rückstand an Kieselgel chromatographiert (Toluol: Ethylacetat 80:20). Ausbeute: 1,3 g (54%);

c) 2-Benzyloxy-1-(2-benzyloxy-phenyl)-ethanon:

Eine Lösung von 0,73 g Benzoesäure in 20 ml wasserfreiem Tetrahydrofuran wird mit 1,23 g N,N-Dicyclohexylcarbodiimid und anschließend tropfenweise mit einer Lösung von 1,3 g 1-(2-Benzyloxy-phenyl)-2-hydroxy-ethanon gemäß Stufe (b) versetzt. Nach abschließender Zugabe von 100 mg N,N-Dimethylaminopyridin wird ca. 12 Stunden bei Raumtemperatur gerührt und der ausgefallene Niederschlag abgetrennt. Das Lösemittel wird im Vakuum abdestilliert und der verbleibende Rückstand an Kieselgel chromatographiert (Toluol-Ethylacetat 80:20). Ausbeute: 0,95 g (51%);

d) 4-(2-Benzyloxyphenyl)-2-phenyl-1,3-oxazol

3.46 g 2-Benzyloxy-1-(2-benzyloxy-phenyl)-ethanon gemäß Stufe (c) werden mit 2,95 g Acetamid und 0,9 ml Bortrifluorid-etherat in 100 ml wasserfreiem Xylol über 20 Stunden zum Rückfluß erhitzt. Nach Abkühlen und Waschen der erhaltenen Lösung mit Wasser wird über Natriumsulfat getrocknet, das Lösemittel im Vakuum abdestilliert und der verbleibende Rückstand an Kieselgel chromatographiert (Toluol). Neben reisoliertem Edukt werden 1 g (31 %) der Zielverbindung isoliert.

e) 4-(2-Hydroxyphenyl)-2-phenyl-1,3-oxazol

0,7 g 4-(2-Benzyloxyphenyl)-2-phenyl-1,3-oxazol gemäß Stufe (d) werden 30 ml Tetrahydrofuran in Gegenwart von 0,1 g Pd/C-Katalysator bei 20-60°C hydriert (5 bar). Nach Abtrennen des Katalysators wird das Lösemittel im Vakuum abdestilliert und der verbleibende Rückstand an Kieselgel chromatographiert (Dioxan:Hexan 1:5). Ausbeute: 0,5 g (99%);

f) 4-{2-[2(N,N-Dimethylamino)ethyl]oxy-phenyl}-2-phenyl-1,3-oxazol

0,48 g 4-(2-Hydroxyphenyl}-2-phenyl-1,3-oxazol werden in 6 ml DMF gelöst und bei 0°C langsam mit 2 ml einer 1 molaren Lösung von Lithiumhexamethyldisilazid in Hexan versetzt. Nach 15 Minuten Rühren bei Raumtemperatur wird tropfenweise eine zuvor 30 Minuten gerührte Mischung aus 0,86 g 2-N,N-Dimethylaminoethylchlorid-hydrochlorid und 6 mmol Lithiumhexamethyldisilazan in 8 ml DMF zugegeben. Diese Mischung wird für 8 Stunden bis zum Rückfluß erhitzt und anschließend im Vakuum vom Lösemittel befreit. Der Rückstand wird in Wasser aufgenommen und zweimal mit Ethylacetat extrahiert. Die organische Phase wird über Natriumsulfat getrocknet, im Vakuum eingeengt und an Kieselgel chromatographiert (Ethylacetat:isopropanol 70:30). Nach der Reinigung wird die Titelverbindung in das Hydrochlorid überführt.
Ausbeute: 0,2 g (29%), Schmp. (Hydrochlorid): 245-246°C;

**Beispiel 9:** 4-{2-[2(N,N-Dimethylamino)ethyl]oxy-phenyl}-2-phenyl-imidazol

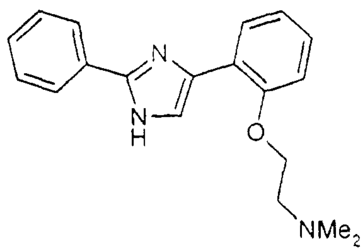

a) 1-(2-Benzyloxy-phenyl)-2-brom-ethanon:

2,9 g 2-Benzyloxyacetophenon (Beispiel 8, Stufe (a)) werden in 50 ml wasserfreiem Chloroform aufgenommen und bei ca. 2°C unter Rühren langsam mit einer Lösung von 2.1 g Brom in 10 ml wasserfreiem Chloroform versetzt. Nach vollständigem Umsatz wird das Lösemittel im Vakuum abdestilliert und der verbleibende Rückstand an Kieselgel chromatographiert (Toluol).
Ausbeute: 2,3 g (59%);

b) 4-(2-Benzyloxyphenyl)-2-phenyl-1,3-imidazol

1,5 g 1-(2-Benzyloxy-phenyl)-2-brom-ethanon gemäß Stufe (a) werden mit 2,4 g Benzamidin-hydrochlorid in 30 ml wasserfreiem Chloroform über 3 Stunden zum Rückfluß erhitzt. Nach Abkühlen und Abdestillieren des Lösemittels im Vakuum wird der Rückstand in Dichlormethan aufgenommen und mit Wasser gewaschen. Die abgetrennt organische Phase wird über Natriumsulfat getrocknet, das Lösemittel im Vakuum abdestilliert und der verbleibende Rückstand an Kieselgel chromatographiert (Dichlormethan:Methanol 98:2). Ausbeute: 1,2 g (82%);

c) 4-(2-Benzyloxyphenyl)-1-tert.butoxycarbonyl-2-phenyl-1,3-imidazol

1,7 g 4-(2-Benzyloxyphenyl)-2-phenyl-1,3-imidazol gemäß Stufe (b) werden mit 0,3 g N,N-Dimethylaminopyridin in 50 ml wasserfreiem Tetrahydrofuran vorgelegt und unter Rühren bei ca. 10°C tropfenweise mit einer Lösung von 2,18 g Di-tert.butyldicarbonat ($BOC_2O$) in 20 ml wasserfreiem Tetrahydrofuran versetzt. Nach 3 Stunden Rühren bei Raumtemperatur und Abdestillieren des Lösemittels im Vakuum wird der Rückstand in Ethylacetat aufgenommen und nacheinander mit $KHSLO_4$-Lösung, $NaHCO_3$-Lösung ung NaCl-Läösung gewaschen. Die abgetrennte organische Phase wird über Natriumsulfat getrocknet und das Lösemittel im Vakuum abdestilliert. Ausbeute: 2,2 g (99%);

d) 1-tert.Butoxycarbonyl-4-(2-hydroxyphenyl)-2-phenyl-1,3-imidazol

2,2 g 4-(2-Benzyloxyphenyl)-1-tert.butoxycarbonyl-2-phenyl-1,3-imidazol gemäß Stufe (c) werden in 35 ml Tetrahydrofuran in Gegenwart von 0,2 g Pd/C-Katalysator bei Raumtemperatur hydriert (5 bar). Nach Abtrennen des Katalysators wird das Lösemittel im Vakuum abdestilliert und der verbleibende Rückstand an Kieselgel chromatographiert (Toluol). Ausbeute:0,75 g (43%);

e) 1-tert.Butoxycarbonyl-4-{2-[2(N,N-dimethylamino)ethyl]oxy-phenyl}-2-phenyl-imidazol

1,15 g 1-tert.Butoxycarbonyl-4-(2-hydroxyphenyl)-2-phenyl-1,3-imidazol werden in 15 ml DMF gelöst und mit 0,136 g Natriumhydrid (60% in Öl) versetzt. Man rührt anschließend noch 30 Minuten bei Raumtemperatur nach und gibt dann eine zuvor 30 Minuten gerührte Mischung aus 0,72 g 2-N,N-Dimethylaminoethylchloridhydrochlorid und 0,2 g Natriumhydrid (60% in Öl) in 20 ml DMF zu. Diese Mischung wird für 2 Stunden auf 100°C erhitzt, und anschließend im Vakuum vom Lösemittel befreit. Der Rückstand wird in Wasser aufgenommen und zweimal mit Dichlormethan extrahiert. Die vereinigten organischen Phasen werden über Natriumsulfat getrocknet, im Vakuum eingeengt und an Kieselgel chromatographiert (Dioxan:Hexan 1:5). Ausbeute: 0,75 g (54%);

f) 4-{2-[2(N,N-Dimethylamino)ethyl]oxy-phenyl}-2-phenyl-imidazol

0,75 g 1-tert.Butoxycarbonyl-4-{2-[2(N,N-dimethylamino)ethyl]oxy-phenyl}-2-phenylimidazol in 5 ml Ethanol werden mit 20 ml einer 2 M Lösung von HCl in Ethylacetat versetzt, 4 Stunden bei Raumtemperatur gerührt und anschließend im Vakuum vom Lösemittel befreit. Der Rückstand wird in Wasser aufgenommen, mit wässriger Ammoniaklösung alkalisch gestellt und zweimal mit Diethylether extrahiert. Die organische Phase wird über Natriumsulfat getrocknet, im Vakuum eingeengt und an Kieselgel chromatographiert (Ethylacetat:Isopropanol 70:30). Nach der Reinigung wird die Titelverbindung in das Dihydrochlorid überführt.
Ausbeute: 0,27 g (39%), Schmp. (Hydrochlorid): 155-158°C;

**Beispiel 10:** 5-{2-[2(N,N-Dimethylamino)ethyl]oxy-phenyl}-2-phenyl-1,3-oxazol

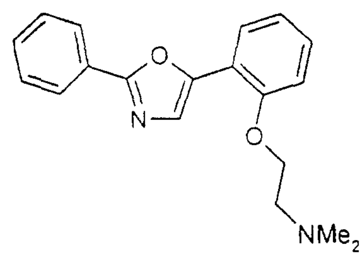

a) 2-Amino-1-(2-benzyloxy-phenyl)-ethanon:

2 g 1-(2-Benzyloxy-phenyl)-2-brom-ethanon (Beispiel 9, Stufe (a)) werden in 15 ml wasserfreiem Chloroform aufgenommen und mit einer Lösung von 1,8 g Urotropin in 40 ml wasserfreiem Chloroform versetzt. Nach 6 Stunden Rühren bei Raumtemperatur wird der ausgefallene Niederschlag abgetrennt und die verbleibende Lösung eingeengt. Nach Abdestillieren des Lösemittels im Vakuum wird der verbleibende Rückstand in Ethanol gelöst, unter Kühlung mit 5 ml konz. Salzsäure versetzt und 12 Stunden bei Raumtemperatur stehen gelassen. Das Lösemittel wird im Vakuum abdestilliert und das erhaltene Rohprodukt ohne weitere Reinigung in die nächste Stufe eingesetzt. Ausbeute: 2,4 g (Rohprodukt).

b) 2-Benzoylamino-1-(2-benzyloxy-phenyl)-ethanon:

1,66 g des Rohprodukts aus Stufe (a) werden in 50 ml wasserfreiem Tetrahydrofuran aufgenommen und nacheinander mit 10 ml Pyridin, 5 ml Dimethylformamid, 5 ml Triethylamin und 2 ml Benzoesäurechlorid versetzt und bei erhöhter Temperatur (ca. 45°C) gerührt. Nach vollständigem Umsatz wird das Lösemittel abdestilliert, der Rückstand in Dichlormethan aufgenommen und die organische Phase mit Wasser gewaschen. Nach Trocknen der organischen Phase über $Na_2SO_4$ wird das Lösemittel im Vakuum abdestilliert und der verbleibende Rückstand an Kieselgel chromatographiert (Toluol:Ethylacetat 90:10). Ausbeute: 0,6 g (30%);

c) 5-(2-Benzyloxyphenyl)-2-phenyl-1,3-oxazol

0,6 g 2-Benzoylamino-1-(2-benzyloxy-phenyl)-ethanon gemäß Stufe (b) werden mit 10 ml Phosphoroxychlorid unter Rühren 2 Stunden auf 110-115°C erwärmt. Nach Abkühlen wird auf Eis gegossen, mit wässriger Ammoniaklösung alkalisch gestellt und zweimal mit Ethylacetat extrahiert.Die vereinigten organischen Phasen werden über Natriumsulfat getrocknet, das Lösemittel wird im Vakuum abdestilliert und der verbleibende Rückstand an Kieselgel chromatographiert (Dioxan:Hexan 1:5). Ausbeute: 0,55 g (96%.);

**Beispiel 11:** 5-(2-Methoxyphenyl)-3-phenyl-1,3,4-oxadiazol-2-on

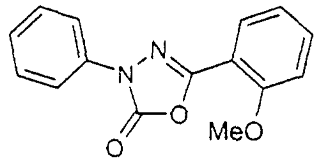

Zu einer Lösung von 6,3 g N'-Methoxybenzoyl-N-phenyl-hydrazin- carbonsäureethylester in wasserfreiem Tetrahydrofuran werden bei Raumtemperatur unter Rühren 3,2 ml Trichloromethylchlorformiat (Diphosgen) gegeben. Nach einer Stunde Rühren bei Raumtemperatur wird das Lösemittel im Vakuum abdestilliert und der verbleibende Rückstand aus Ethanol umkristallisiert. Ausbeute: 6,5 g (96%), Schmp.: 112-115°C;

**Beispiel** 12: 5-{2-[2(N,N-Dimethylamino)ethyl]oxy-phenyl}-3-phenyl-1,3,4-oxadiazol-2-on

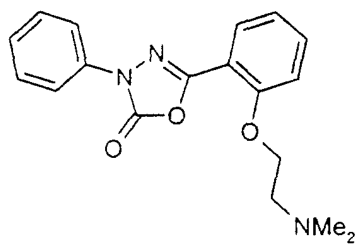

a) 5-(2-Hydroxyphenyl)-3-phenyl-1,3,4-oxadiazol-2-on:

6,4 g 5-(2-Methoxyphenyl)-3-phenyl-1,3,4-oxadiazol-2-on (Beispiel 11 ) werden in 60 ml Dichlormethan gelöst und unter Kühlung (0-5°C) tropfenweise mit 17,9 g $BBr_3$ versetzt. Nach 24 Stunden Rühren bei Raumtemperatur wird der Ansatz vorsichtig auf 50 ml gekühltes Methanol gegeben und mit verdünnter, wässriger HCl-Lösung versetzt. Die organische Phase wird abgetrennt und nacheinander mit wässriger $Na_2CO_3$-Lösung (17%-ig) und Wasser gewaschen. Nach Trocknen der gesammelten organischen Phasen über $Na_2SO_4$ wird das Lösemittel abdestilliert. Das verbleibende Rohprodukt wird durch Kristallisation aus n-Hexan gereinigt. Ausbeute: 5,2 g (86%), Schmp.: 148°C;

b) 5-{2-[2(N,N-Dimethylamino)ethyl]oxy-phenyl}-3-phenyl-1,3,4-oxadiazol-2-on

1 g 5-(2-Hydroxyphenyl)-3-phenyl-1,3,4-oxadiazol-2-on werden in 10 ml DMF gelöst und mit 0,24 g Natriumhydrid (80% in Öl) versetzt. Man rührt anschließend noch 20 Minuten bei Raumtemperatur und gibt dann 0,57 g 2-N, N-Dimethylaminoethylchloridhydrochlorid zu. Diese Mischung wird für 3 Stunden auf 100°C erhitzt und nach Abkühlen auf Wasser gegeben. Nach Extraktion mit Essigsäureethylester und Trocknen der vereinigten organischen Phasen über Natriumsulfat wird das Lösemittel im Vakuum abdestilliert und der verbleibende Rückstand an Kieselgel chromatographiert (Dichlormethan:Methanol 95:5). Die so erhaltene freie Base wird in Aceton gelöst und durch Zugabe von ethanolischer HCl-Lösung in das Hydrochlorid überführt. Ausbeute: 0,34 g, Schmp.: 170°C (Hydrochlorid);

**Beispiel 13:** 3-(2-Methoxyphenyl)-5-phenyl-1H-pyrrol

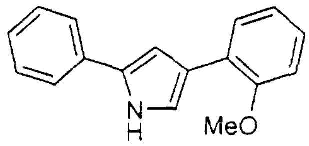

a) 3-(2-Methoxyphenyl)-1-phenyl-2-propen-1-on:

Zu einer Lösung von 6,4 g NaOH in 90 ml Wasser und 40 ml Ethanol werden bei 10°C 15 g Acetophenon und 17 g 2-Methoxybenzaldehyd gegeben. Nach 5 Stunden Rühren bei Raumtemperatur wird mit Wasser verdünnt und mit Dichlormethan extrahiert. Die organische Phase wird über $MgSO_4$ getrocknet und eingeengt. Es verbleiben 32 g 3-(2-Methoxyphenyl)-1 -phenyl-2-propen-1-on als farbloses Öl, das ohne weitergehende Reinigung in die nächste Stufe eingesetzt wird.

b) 3-(2-Methoxyphenyl)-4-nitro-1-phenyl-butan-1-on:

Zu einer Lösung von 32 g 3-(2-Methoxyphenyl)-1-phenyl-2-propen-1-on und 6,6 ml Nitromethan in Methanol werden 3,3 g Natriummethylat in Methanol gegeben. Die erhaltene Mischung wird unter Rühren 2 Stunden auf 50°C erhitzt und nach Abkühlen durch Zugabe von Essigsäure langsam auf pH 5 eingestellt. Diese Lösung wird mit Wasser verdünnt und anschließend mit Dichlormethan extrahiert. Nach Trocknen der gesammelten organischen Phasen wird das Lösemittel abdestilliert. Es verbleiben 34 g 3-(2-Methoxyphenyl)-4-nitro-1-phenyl-butan-1-on als farbloses Öl, das ohne weitergehende Reinigung in die nächste Stufe eingesetzt wird.

c) 3-(2-Methoxyphenyl)-5-phenyl-2,3-dihydro-1H-pyrrol:

34 g 3-(2-Methoxyphenyl)-4-nitro-1-phenyl-butan-1-on werden mit Raney-Nickel in Ethylacetat für 2 Tage unter einer Wasserstoffatmosphäre gerührt. Nach Abfiltrieren des Katalysators und Abdestillieren des Lösemittels im Vakuum verbleiben 24 g 3-(2-Methoxyphenyl)-5-phenyl-2,3-dihydro-1H-pyrrol als farbloses Öl, das ohne weitergehende Reinigung in die nächste Stufe eingesetzt wird.

d) 3-(2-Methoxyphenyl)-5-phenyl-1H-pyrrol:

Eine Mischung von verbleiben 24 g 3-(2-Methoxyphenyl)-5-phenyl-2,3-dihydro-1Hpyrrol und 2 g Pd/C (30%-ig) in 200 ml p-Cymol wird für 2 Stunden zum Rückfluß erhitzt. Nach Abfiltrieren des Katalysators und Abdestillieren des Lösemittels im Vakuum verbleit ein öliger Rückstand, der durch Chromatographie an Kieselgel (Cyclohexan:Ethylacetat 9:1) gereinigt wird.
Ausbeute: 6 g (25%), Schmp.: 90-93 °C (n-Hexan);

**Beispiel 14:** 3-{2-[2(N,N-Dimethylamino)ethyl]oxy-phenyl}-5-phenyl-1H-pyrrol

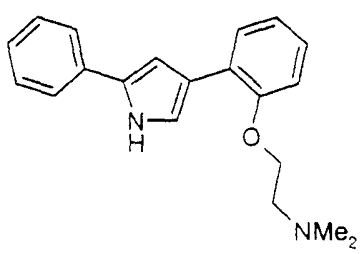

a) 3-(2-Hydroxyphenyl)-5-phenyl-1H-pyrrol:

Die Darstellung erfolgte ausgehend von 3-(2-Methoxyphenyl)-5-phenyl-1H-pyrrol (Beispiel 3) in Analogie zu Beispiel 12 (Stufe a). Schmp.: 144-146°C;

b) 3-{2-[2(N,N-Dimethylamino)ethyl]oxy-phenyl}-5-phenyl-1H-pyrrol:

Die Darstellung erfolgte ausgehend von 3-(2-Hydroxyphenyl)-5-phenyl -1H-pyrroi in Analogie zu Beispiel 12 (Stufe b). Schmp.: 105-108°C;

**Beispiel 15:** 1-(2-Methoxyphenyl)-3-phenyl-pyrrol

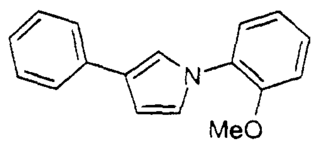

a) 2-(2-Methoxyphenyl)amino-1-phenyl-ethan-1-on:

Eine Mischung von 10 g 2-Bromacetophenon, 5,62 ml 2-Methoxyanilin und 5,85 g $Na_2CO_3$ in Ethanol wird 18 Stunden gerührt. Nach Verdünnen mit Wasser wird weitere 30 Minuten gerührt und die Mischung filtriert. Nach Waschen des abgetrennten Feststoffs mit Wasser und Hexan verbleiben 9,7 g 2-(2-Methoxyphenyl)amino-1-phenyl-ethan-1-on als grün-gelber Feststoff. Eine weitergehende Reinigung erfolgt nicht.

b) 2,3-Di(methoxycarbonyl)-1-(2-methoxyphenyl)-4-phenyl-pyrrol:

Eine Lösung von 7,4 g 2-(2-Methoxyphenyl)amino-1-phenyl-ethan-1-on und 11,3 ml Acetylendicarbonsäuredimethylester in 150 ml Toluol wird 12 Stunden zum Rückfluß erhitzt und anschließend im Vakuum vom Lösemittel befreit. Der verbleibende Rückstand wird in Ethylacetat aufgenommen und mit Wasser gewaschen. Nach Trocknen der organischen Phase wird das Lösemittel im Vakuum abdestilliert und der verbleibende Rückstand an Kieselgel chromatographiert (n-Hexane:Ethylacetat 80:20). Man erhält 9,4 g 2,3-Di(methoxycarbonyl)-1-(2-methoxyphenyl)-4-phenylpyrrol als farbloses Öl.

c) 2,3-Dicarboxy-1-(2-methoxyphenyl)-4-phenyl-pyrrol:

Eine Lösung von 8,4 g 2,3-Di(methoxycarbonyl)-1-(2-methoxyphenyl)-4-phenyl-pyrrol und 2,6 g KOH in Diethylenglycolmonomethylether wird für 3 Stunden um Rückfluß erhitzt. Nach Abkühlen wird auf Wasser gegeben, angesäuert und 3 mal mit Diethylether extrahiert. Die vereinigten organischen Phasen werden mit Wasser gewaschen, getrocknet und eingeengt. Das Rohprodukt wird aus Diethylether kristallisiert. Es werden 4,8 g 2,3-Dicarboxy-1-(2-methoxyphenyl)-4-phenyl-pyrrol erhalten.

d) 1-(2-Methoxyphenyl)-3-phenyl-pyrrol:

In 50 ml Chinolin werden 4,8 g 2,3-Dicarboxy-1-(2-methoxyphenyl)-4-phenyl-pyrrol und 2 g Cu-Pulver für 2 Stunden auf ca. 200°C erhitzt. Nach dem Abkühlen wird das Cu-Pulver abfiltriert. Das Filtrat wird mit Wasser verdünnt, mit verdünnter wässriger HCl-Lösung angesäuert und mit Ethylacetat extrahiert. Die organische Phase wird mit nacheinander mit verdünnter, wässriger HCl-Lösung, $NaHCO_3$-Lösung und Wasser gewaschen und anschließend getrocknet. Nach Abdestillieren des Lösemittels im Vakuum wird durch Chromatographie an Kieselgel gereinigt (Cyclohexan: Ethylacetat 95:5). Es werden 1,6 g 1-(2-Methoxyphenyl)-3-phenyl-pyrrol in Form eines farblosen Öls erhalten.

**Beispiel 16:** 1-{2-[2(N,N-Dimethylamino)ethyl]oxy-phenyl}-3-phenyl-pyrrol

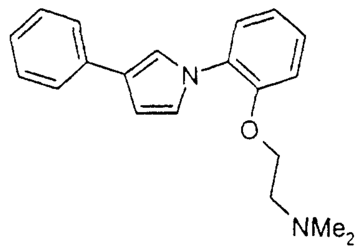

a) 1-(2-Hydroxyphenyl)-3-phenyl-pyrrol:

Die Darstellung erfolgte ausgehend von 1-(2-Methoxyphenyl)-3-phenyl-pyrrol (Beispiel 15) in Analogie zu Beispiel 12 (Stufe a). farbloses Öl;

b) 1-{2[2(N,N-Dimethylamino)ethyl]oxy-phenyl}-3-phenyl-pyrrol:

Die Darstellung erfolgte ausgehend von 1-(2-Hydroxyphenyl)-3-phenyl-pyrrol in Analogie zu Beispiel 12 (Stufe b). Schmp.: 116-121°C (Hydrochlorid);

**Beispiel 17:** 4-(2-Methoxyphenyl)-1-phenyl-pyrazol

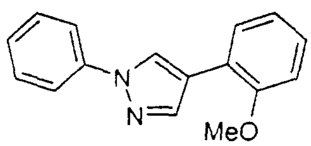

a) 1-Methoxy-2-(2-methylsulfanyl-vinyl)-benzol:

Zu einer Lösung von 1,5 g Methylmercaptomethyl-triphenyl-phosphoniumchlorid in wasserfreiem Tetrahydrofuran werden bei 0°C langsam 2,6 ml einer 1,6 M Buthyllithium-Lösung in Tetrahydrofuran zugetropft. Nach 1 Stunde werden 5,7 g 2-Methoxybenzaldehyd in 50 ml Tetrahydrofuran zugegeben und es wird weitere 12 Stunden bei Raumtemperatur gerührt. Das Lösemittel wird im Vakuum abdestilliert und der verbeleibende Rückstand in Diethylether aufgenommen. Nach Extrahieren mit verdünnter, wässriger HCl-Lösung, wässriger $Na_2CO_3$-Lösung (17%) und Waschen mit Wasser werden die gesammelten organischen Phasen getrocknet und das Lösemittel im Vakuum abdestilliert. Chromatographie an Kieselgel (Cyclohexan:Ethylacetat 90:10) liefert 5,5 g 1-Methoxy-2-(2-methylsulfanyl-vinyl)-benzol.

b) 2-(2-Methoxyphenyl)-3-methylsulfanyl-propenal:

Eine Lösung von 5,5 g 1-Methoxy-2-(2-methylsulfanyl-vinyl)-benzol in 15 ml Dimethylformamid wird mit 4,7 g $POCl_3$ unter Rühren für 2 Stunden auf 80°C erhitzt. Nach Abkühlen wird vorsichtig Diethylether und Wasser zugesetzt und die erhaltene Mischung mit wässriger NaOH-Lösung (10%) auf pH 9 eingestellt. Die organische Phase wird abgetrennt und nach Trocknen im Vakuum vom Lösemittel befreit. Chromatographie an Kieselgel (Cyclohexan:Ethylacetat 70:30) liefert 2,5 g 2-(2-Methoxyphenyl)-3-methylsulfanyl-propenal.

c) 4-(2-Methoxyphenyl)-1-phenyl-pyrazol:

Eine Lösung von 2,4 g 2-(2-Methoxyphenyl)-3-methylsulfanyl-propenal in wasserfreiem Dioxan wird mit 1,3

g Phenylhydrazin über 24 Stunden zum Rückfluß erhitzt. Das Lösemittel wird im Vakuum abdestilliert und der Rückstand in Diethylether und verdünnter, wässriger HCl-Lösung aufgenommen. Die organische Phase wird getrocknet und eingeengt, der ölige Rückstand an Kieselgel (Cyclohexan:Ethylacetat 95:5) gereinigt.
Ausbeute: 0,85 g, Schmp.: 60-68°C (n-Hexan);

**Beispiel 18:** 4-{2-[2(N,N-Dimethylamino)ethyl]oxy-phenyl}-1-phenyl-pyrazol

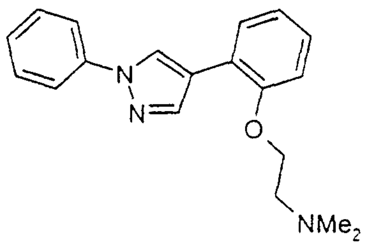

a) 4-(2-Hydroxyphenyl)-1-phenyl-pyrazol:

Die Darstellung erfolgte ausgehend von 4-(2-Methoxyphenyl)-1-phenyl-pyrazol (Beispiel 17) in Analogie zu Beispiel 12 (Stufe a). Schmp.: 205-208°C (Diethylether);

b) 4-{2-[2(N,N-Dimethylamino)ethyl]oxy-phenyl}-1-phenyl-pyrazol:

Die Darstellung erfolgte ausgehend von 4-(2-Hydroxyphenyl)-1-phenyl-pyrazol in Analogie zu Beispiel 12 (Stufe b). Schmp.: 150°C (Hydrochlorid);

**Beispiel 19:** 3-{2-[2(N,N-Dimethylamino)ethyl]oxy-phenyl}-5-phenyl-imidazolidin-2,4-dion

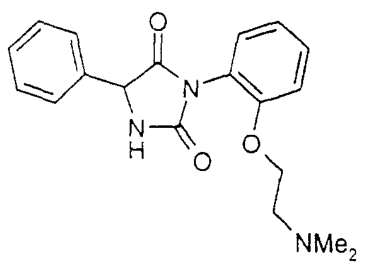

a) 1-[2(N,N-Dimethylamino)ethyl]oxy-2-nitro-benzol:

Zu 24 g Nitrophenol und 72 g $K_2CO_3$ in wasserfreiem Aceton werden unter Rühren 23 g 2-N,N-Dimethylaminoethylchlorid-hydrochlorid gegeben. Diese Mischung wird 7 Stunden unter Rückfluß erhitzt. Zur Aufarbeitung wird abgekühlt und langsam mit verdünnter, wässriger HCl-Lösung versetzt. Nach Extraktion mit Diethylether wird die wässrige Phase auf pH 9 eingestellt und mit Ethylacetat extrahiert. Trocknen der Ethylacetatphase und Abdestillieren des Lösemittels im Vakuum liefern 11 g 1-[2(N,N-Dimethylamino)ethyl]oxy-2-nitro-benzol als gelbes Öl, welches ohne weitere Reinigung in die nächste Phase eingesetzt wird.

b) 2-[2(N,N-Dimethylamino)ethyl]oxy-anilin:

in 200 ml Ethanol werden 11 g 1-[2(N,N-Dimethylamino)ethyl]oxy-2-nitro-benzol bei Raumtemperatur über einen Zeitraum von 6 Stunden in Gegenwart von 0,5 g Pd/C (10%) unter einer Wasserstoffatmosphäre gerührt. Nach Filtration und Abdestillieren des Lösemittels im Vakuum verbleiben 8,5 g eines farblosen Öls, welches ohne weitere Reinigung in die nächste Stufe eingesetzt wird.

c) 1-[2(N,N-Dimethylamino)ethyl]oxy-2-isocyanat-benzol:

Zu 8 g 2-[2(N,N-Dimethylamino)ethyl]oxy-anilin in 80 ml Dioxan werden 5,5 ml Trichlormethyl-chlorformiat (Diphosgen) gegeben. Nach 2 Stunden Rühren bei 60°C wird das Lösemittel im Vakuum abdestilliert. Es verbleiben 9 g Rohprodukt, welches ohne weitere Reinigung in die nächste Stufe eingesetzt wird.

d) 3-{2-[2(N,N-Dimethylamino)ethyl]oxy-phenyl}-5-phenyl-imidazolidin-2,4-dion

Zu einer Lösung von 6,6 g Phenylglycin und 2,9 g Kaliumhydroxid in Wasser (ca. 20 ml) werden 10 g des gemäß Stufe (c) generierten Isocyanats langsam zugesetzt. Die Mischung wird 12 Stunden bei Raumtemperatur gerührt, filtriert und mit verdünnter, wässriger HCl-Lösung angesäuert (ca. pH 2). Der gebildete Niederschlag wird abgetrennt und in einer Mischung von 8%-iger wässriger HCl-Lösung in Ethanol (150 ml) zum Rückfluß erhitzt. Nach 6 Stunden wird langsam abgekühlt und mit wässriger $Na_2CO_3$-Lösung (17%-ig) alkalisch gestellt. Der erhaltene Niederschlag wird aus Ethylacetat umkristallisiert. Es werden 2,4 g der Titelverbindung erhalten.

**Beispiel 20:** 1-{2-[2(N,N-Dimethylamino)ethyl]oxy-phenyl}-4-phenyl-1,3-dihydroimidazol-2-on

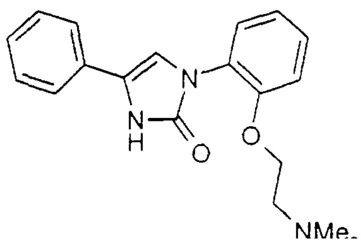

Zu einer Lösung von 2,4 g 3-{2-[2(N,N-Dimethylamino)ethyl]oxy-phenyl}-5-phenylimidazolidin-2,4-dion (siehe Beispiel 9) in 50 ml wasserfreiem Tetrahydrofuran werden langsam 14,1 ml RED-Al zugegeben. Diese Mischung wird 12 Stunden bei Raumtemperatur gerührt, abgekühlt und mit wässriger HCl-Lösung (10%-ig) versetzt. Nach Extraktion mit Dichlormethan, Trocknen der gesammelten organischen Phasen und Abdestillieren des Lösemittels im Vakuum wird durch Chromatographie an Kieselgel gereinigt (Dichlormethan:Methanol 90:10). Das so erhaltene Öl wird mittels etherischer HCl-Lösung in das Hydrochlorid überführt.
Ausbeute: 0,4 g; Schmp.: 220-223°C (Hydrochlorid).

**Beispiel 21:** 5-{2-[2(N,N-Dimethylamino)ethyl]oxy-phenyl}-3-phenyl-imidazolidin-2,4-dion

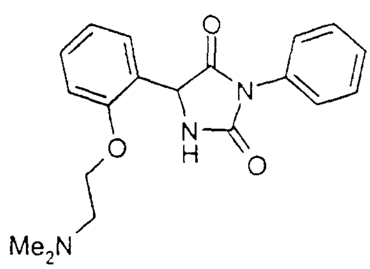

a) 2-[2(N,N-Dimethylamino)ethyl]oxy-benzaldehyd:

Zu 26 ml 2-Hydroxybenzaldehyd und 90 g $K_2CO_3$ in wasserfreiem Aceton werden unter Rühren 58 g 2-N, N-Dimethylaminoethylchlorid-hydrochlorid gegeben. Diese Mischung wird 7 Stunden unter Rückfluß erhitzt. Zur Aufarbeitung wird abgekühlt und langsam mit verdünnter, wässriger HCl-Lösung (5%-ig) versetzt. Nach Extraktion mit Diethylether wird die wässrige Phase auf pH 9 eingestellt und mit Ethylacetat extrahiert. Trocknen der Ethylacetatphase und Abdestillieren des Lösemittels im Vakuum liefern 11 g 2-[2(N,N-Dimethylamino)ethyl]oxy-benzaldehyd als gelbes

Öl, welches ohne weitere Reinigung in die nächste Phase eingesetzt wird.

b) 2-Amino-2-{[2(N,N-dimethylamino)ethyl]oxy-phenyl}-essigsäure:

In 500 ml Ethanol (50%-ig) werden 29 g 2-[2(N,N-Dimethylamino)ethyl]oxybenzaldehyd-hydrochlorid und 73 g Ammoniumcarbonat aufgenommen. Diese Mischung wird mit 16,4 g Kaliumcyanid versetzt. Diese Mischung wird auf 60°C erwärmt und bei konstanter Temperatur 8 Stunden gerührt. Nach Abkühlen wird mit wässriger HCl auf pH 2 angesäuert. Das Ethanol wird vorsichtig im Vakuum abdestilliert und die wässrige Phase wird mit wässriger NaOH-Lösung (10%-ig) alkalisch gestellt (pH 10). Nach Extraktion mit Dichlormathan und Abdestillieren des organischen Lösemittels verbleiben 10 g eines weißen Niederschlags, der in wässriger HBr-Lösung (48%-ig) 14 Stunden zum Rückfluß erhitzt wird. Das Produkt (5 g) wird in Form eines weißen Feststoffs (Schmp.: 195-196°C) nach Einengen und Kristallisation aus Methanol:Essigsäure 50:1 erhalten.

c) 5-{2-[2(N,N-Dimethylamino)ethyl]oxy-phenyl}-3-phenyl-imidazolidin-2,4-dion

Zu einer Lösung von 4 g 2-Amino-2-{[2(N,N-dimethylamino)ethyl]oxy-phenyl}essigsäure und 1,65 g Kaliumhydroxid in Wasser (ca. 20 ml) werden langsam 1,4 ml Phenylisocyanat zugesetzt. Die Mischung wird 12 Stunden bei Raumtemperatur gerührt, filtriert, mit wässriger HCl-Lösung (10%-ig) angesäuert (ca. pH 2) und zum Rückfluß erhitzt. Nach 6 Stunden wird langsam abgekühlt und mit wässriger $Na_2CO_3$-Lösung (10%-ig) alkalisch gestellt. Der erhaltene Niederschlag wird aus Ethylacetat umkristallisiert. Es werden 3 g der Titelverbindung in Form eines weißen Feststoffs (Schmp.: 120-124°C) erhalten.

**Beispiel 22:** 4-{2-[2(N,N-Dimethylamino)ethyl]oxy-phenyl}-1-phenyl-1,3-dihydroimidazol-2-on

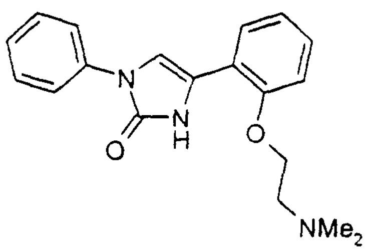

Zu einer Lösung von 1,5 g 5-{2-[2(N,N-Dimethylamino)ethyl]oxy-phenyl}-3-phenylimidazolidin-2,4-dion (siehe Beispiel 11 ) in 30 ml wasserfreiem Tetrahydrofuran werden langsam 8,8 ml RED-Al zugegeben. Diese Mischung wird 12 Stunden bei Raumtemperatur gerührt, abgekühlt und mit wässriger HCl-Lösung (10%-ig) versetzt. Nach Extraktion mit Dichlormethan, Trocknen der gesammelten organischen Phasen und Abdestillieren des Lösemittels im Vakuum wird durch Chromatographie an Kieselgel gereinigt (Dichlormethan:Methanol 90:10). Das so erhaltene Öl wird mittels etherischer HCl-Lösung in das Hydrochlorid überführt.
Ausbeute: 0,15 g; Schmp.: 215-218°C (Hydrochlorid).

d) 5-(2-Hydroxyphenyl)-2-phenyl-1,3-oxazol

0,55 g 5-(2-Benzyloxyphenyl)-2-phenyl-1,3-oxazol gemäß Stufe (c) werden in 30 ml Tetrahydrofuran in Gegenwart von 0,1 g Pd/C-Katalysator (10%) bei ca. 60°C hydriert (5,7 bar). Nach Abtrennen des Katalysators wird das Lösemittel im Vakuum abdestilliert und der verbleibende Rückstand an Kieselgel chromatographiert (Dioxan:Hexan 3:5). Ausbeute: 0,38 g (95%);

e) 5-{2-[2(N,N-Dimethylamino)ethyl]oxy-phenyl}-2-phenyl-1,3-oxazol

0,38 g 5-(2-Hydroxyphenyl)-2-phenyl-1,3-oxazol werden in 6 ml DMF gelöst und bei 0°C langsam mit 1,6 ml einer 1 molaren Lösung von Lithiumhexamethyldisilazid in Hexan versetzt. Nach 15 Minuten Rühren bei Raumtemperatur wird tropfenweise eine zuvor 30 Minuten gerührte Mischung aus 0,69 g 2-N,N-Dimethylaminoethylchlorid-hydrochlorid und 4,8 mmol Lithiumhexamethyldisilazan in 8 ml DMF zugegeben. Diese Mischung wird für 6 Stunden bis zum Rückfluß erhitzt und anschließend im Vakuum vom Lösemittel befreit. Der Rückstand wird in Wasser aufgenommen

und zweimal mit Ethylacetat extrahiert. Die organische Phase wird über Natriumsulfat getrocknet, im Vakuum eingeengt und an Kieselgel chromatographiert (Ethylacetat:Isopropanol 70:30). Nach der Reinigung wird die Titelverbindung in das Hydrochlorid überführt.
Ausbeute: 0,18 g (33%), Schmp. (Hydrochlorid): 246-247°C;

Gemäß den zuvor beschriebenen Verfahren oder in Anlehnung an Analogieverfahren können ferner die in Tabelle 1 aufgeführten Verbindungen der allgemeinen Formel (I) mit $R^2$, $R^3$, $R^4$ und $R^5$ = Wasserstoff hergestellt werden:

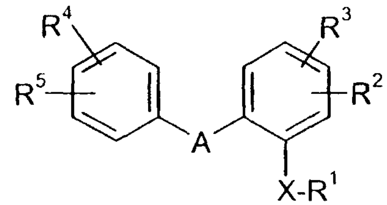

(I)

Tabelle 1:

| Beispiel | 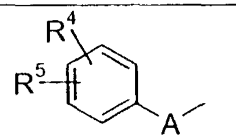 | -X- | $-R^1$ | Schmp. [°C] | Chemische Bezeichnung |
|---|---|---|---|---|---|
| 23 | 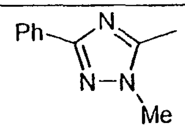 | -O- | 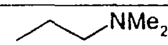 | 118-120 [d] | 5-{2-[2(N,N-Dimethylamino)ethyl]oxy-phenyl}-1-methyl-3-phenyl-1,2,4-triazol |
| 24 | 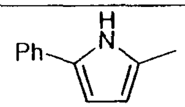 | -O- | 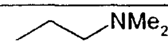 | 237 [a] | 2-{2-[2(N,N-Dimethylamino)ethyl]oxy-phenyl}-5-phenyl-pyrrol |
| 25 | 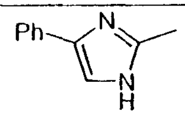 | -O- | 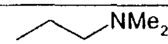 | 176-177 [e] | 2-{2-[2(N,N-Dimethylamino)ethyl]oxy-phenyl}-4-phenyl-imidazol |
| 26 | 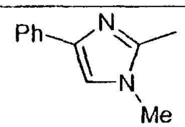 | -O- | 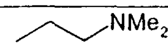 | 141-143 [e] | 2-{2-[2(N,N-Dimethylamino)ethyl]oxy-phenyl}-1-methyl-4-phenyl-imidazol |

| Beispiel | 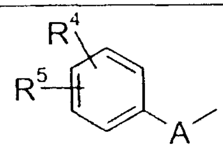 | -X- | $-R^1$ | Schmp. [°C] | Chemische Bezeichnung |
|---|---|---|---|---|---|
| 27 | 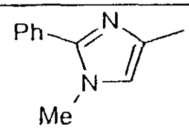 | -O- | 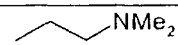 | 177-178 b | 4-{2-[2(N,N-Dimethylamino)ethyl]oxy-phenyl}-1-methyl-2-phenyl-imidazol |
| 28 | 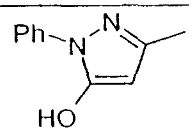 | -O- | 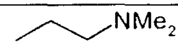 | 185-188 a | 3-{2-[2(N,N-Dimethylamino)ethyl]oxy-phenyl}-5-hydroxy-1-phenyl-pyrazol |
| 29 | 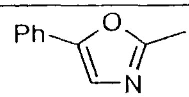 | -O- | 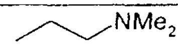 | 178-180 a | 2-{2-[2(N,N-Dimethylamino)ethyl]oxy-phenyl}-5-phenyl-oxazol |
| 30 | 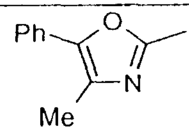 | -O- | 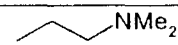 | 155-157 b | 2-{2-[2(N,N-Dimethylamino)ethyl]oxy-phenyl}-4-methyl-5-phenyl-oxazol |
| 31 | 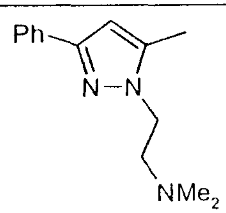 | -O- | -H | 215-217 a | 1-[2(N,N-Dimethylamino)ethyl]-5-(2-hydroxyphenyl)-3-phenyl-pyrazol |
| 32 | 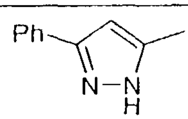 | -O- | 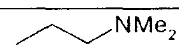 | 130-133 c | 5-{2-[2(N,N-Dimethylamino)ethyl]oxy-phenyl}-3-phenyl-pyrazol |

| Beispiel | 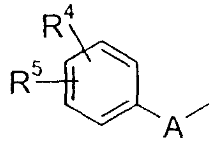 | -X- | $-R^1$ | Schmp. [°C] | Chemische Bezeichnung |
|---|---|---|---|---|---|
| 33 | 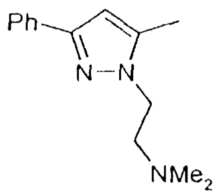 | -O- | 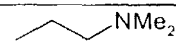 | 253-254 b | 1-[2(N,N-Dimethylamino)ethyl]-5-{2-[2(N,N-dimethylamino)ethyl]oxy-phenyl}-3-phenyl-pyrazol |
| 34 | 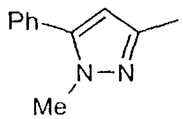 | -O- | 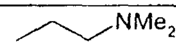 | 218-220 b | 3-{2-[2(N,N-Dimethylamino)ethyl]oxy-phenyl}-1-methyl-5-phenyl-pyrazol |
| 35 | 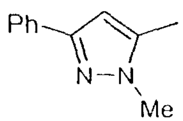 | -O- | 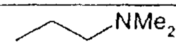 | 113-115 b | 5-{2-[2(N,N-Dimethylamino)ethyl]oxy-phenyl}-1-methyl-3-phenyl-pyrazol |
| 36 | 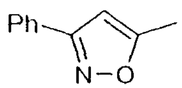 | -O- | 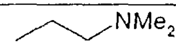 | 182-183 a | 5-{2-[2(N,N-Dimethylamino)ethyl]oxy-phenyl}-3-phenyl-isoxazol |
| 37 | 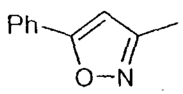 | -O- | 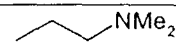 | 172-175 a | 3-{2-[2(N,N-Dimethylamino)ethyl]oxy-phenyl}-5-phenyl-isoxazol |
| 38 | 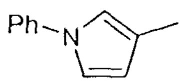 | -O- | -Methyl | 93-95 | 3-(2-Methoxyphenyl)-1-phenyl-pyrrol |
| 39 | 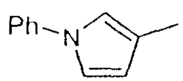 | -O- | -H | 72-74 | 3-(2-Hydroxyphenyl)-1-phenyl-pyrrol |
| 40 | 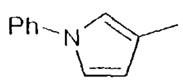 | -O- | 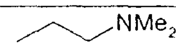 | 217-219 a | 3-{2-[2(N,N-Dimethylamino)ethyl]oxy-phenyl}-1-phenyl-pyrrol |

| Beispiel | 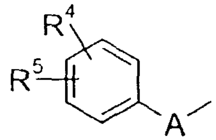 | -X- | $-R^1$ | Schmp. [°C] | Chemische Bezeichnung |
|---|---|---|---|---|---|
| 41 | 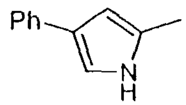 | -O- | 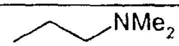 | 177-179 [a] | 2-{2-[2(N,N-Dimethylamino)ethyl]oxy-phenyl}-4-phenyl-1H-pyrrol |
| 42 | 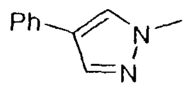 | -O- | -H | - | 1-(2-Hydroxyphenyl)-4-phenyl-pyrazol |
| 43 | 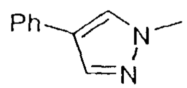 | -O- | -Methyl | - | 1-(2-Methoxyphenyl)-4-phenyl-pyrazol |
| 44 | 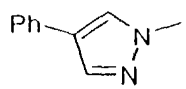 | -O- | 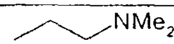 | - | 1-{2-[2(N,N-Dimethylamino)ethyl]oxy-phenyl}-4-phenyl-pyrazol |
| 45 | 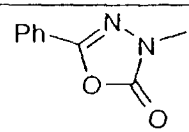 | -O- | -Methyl | 129-131 | 3-(2-Methoxyphenyl)-5-phenyl-1,3,4-oxadiazol-2-on |
| 46 | 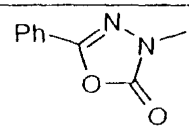 | -O- | -H | 135 | 3-(2-Hydroxyphenyl)-5-phenyl-1,3,4-oxadiazol-2-on |
| 47 | 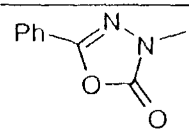 | -O- | 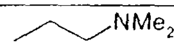 | - | 3-{2-[2(N,N-Dimethylamino)ethyl]oxy-phenyl}-5-phenyl-1,3,4-oxadiazol-2-on |
| 48 | 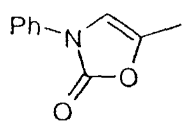 | -O- | -H | 208-209 | 5-(2-Hydroxyphenyl)-3-phenyl-oxazolin-2-on |

| Beispiel | 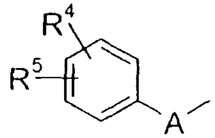 | -X- | -R[1] | Schmp. [°C] | Chemische Bezeichnung |
|---|---|---|---|---|---|
| 49 | 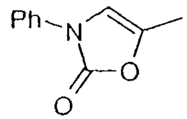 | -O- | -Methyl | 125-127 | 5-(2-Methoxyphenyl)-3-phenyl-oxazolin-2-on |
| 50 | 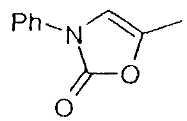 | -O- | 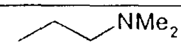 | 205 [a] | 5-{2-[2(N,N-Dimethylamino)ethyl]oxy-phenyl}-3-phenyl-oxazolin-2-on |
| 51 | 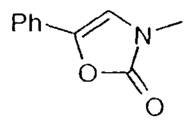 | -O- | -H | 192-193 | 3-(2-Hydroxyphenyl)-5-phenyl-oxazolin-2-on |
| 52 | 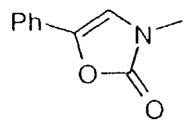 | -O- | -Methyl | 156-158 | 3-(2-Methoxyphenyl)-5-phenyl-oxazolin-2-on |
| 53 | 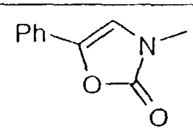 | -O- | 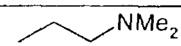 | 180 [a] | 3-{2-[2(N,N-Dimethylamino)ethyl]oxy-phenyl}-5-phenyl-oxazolin-2-on |
| 54 | 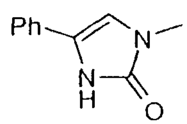 | -O- | -H | 253-255 | 1-(2-Hydroxyphenyl)-4-phenyl-1,3-dihydro-imidazol-2-on |

a) x HCl; b) x 2Hcl; c) x 1,5 HCl; d) x 1,5 Oxalsäure; e) x 2 Oxalsäure;

Überraschenderweise wurde gefunden, daß die erfindungsgemäßen Verbindungen eine Affinität zu oder Aktivität an verschiedenen Rezeptortypen zeigen und eine neuroprotektive Wirkung aufweisen.

*In vitro* und *in vivo* Versuche haben gezeigt, daß die im Gehirn infolge von Hypoglykämie, Hypoxie, Anoxie, globale und fokale Ischämie, Schädel-Hirn-Trauma, Hirn-Ödem und Hirn-Druck auftretenden Zellschäden und Funktionsausfälle z. T. auf einer erhöhten synaptischen Aktivität und somit vermehrter Transmitterfreisetzung beruhen. Neben Glutamat sind Histamin und Serotonin als Neurotransmitter von besonderer Bedeutung. Darüberhinaus werden die Konzentrationen von insbesondere Calcium und Natrium Ionen verändert.

Es ist bekannt, daß nach systemischer Applikation von Glutamat Neuronen im Gehirn von Mäusen zerstört werden (S.M. Rothman und T.W. Olney, Trends in Neurosciences 10 (1987) 299). Dieser Befund läßt unter anderem den Schluß zu, daß Glutamat eine Rolle bei neurodegenerativen Erkrankungen spielt (R.Schwarcz und B. Meldrum, The Lancet 11 (1985) 140). Weiterhin sind Substanzen wie z.B. Quisqualinsäure, Kaininsäure, Ibotensäure, Glutaminsäure, N-Methyl-Dasparaginsäure (NMDA) und α-Amino-3-hydroxy-5-methyl-4-isooxazol-propionsäure (AMPA) als exogene bzw. endogene Neurotoxine bekannt. Gehirnläsionen, die mit solchen Substanzen induziert werden können, sind vergleichbar mit jenen, welche mit Zusammenhang mit Epilepsie und anderen neurodegenerativen Erkrankungen - wie z.B. Morbus Huntington und Morbus Alzheimer - auftreten. Substanzen und Ionen, welche die Aktivität der Glutamat-Rezeptoren und des mit diesem Rezeptor verbundenen Ionenkanals hemmen - wie z.B. kompetitive und nichtkompetitive Antagonisten exzitatorischer Aminosäuren - schützen Gehirnzellen vor hypoxischen bzw. ischämischen Schäden. Diese Befunde zeigen, daß die Glutamat-Rezeptoren eine wichtige Rolle bei der Vermittlung des ischämischen Schadens spielen.

Der Nachweis der Wirkung am AMPA Rezeptor wurde mittels Elektrophysiologie an neuronalen Zellen (Patch-Clamp-Methode) geführt (M. L. Mayer, L. Vyklicky and G. L. Westbrook, J. Physiol. 415 (1989) 329-350).
Die Testung erfolgte bei einer Testkonzentration von 100 μM.

Tabelle 2:

| Hemmung des Kainat-induzierten Signals am AMPA-Rezeptor | |
|---|---|
| Beispiel | AMPA Inh. [%] |
| 1 | 56 |
| 2 | 96 |
| 3 | 74 |
| 5 | 76 |
| 7 | 39 |
| 8 | 77 |
| 10 | 98 |
| 17 | 53 |

Der Nachweis der Affinität zur "$Na^+$ Kanal site 2"-Bindungsstelle wurde wie von G.B. Brown (J. Neurosci. 6 (1986) 2064) beschrieben geführt. Die Testung erfolgte typischerweise bei einer Testkonzentration von 10μM.

Tabelle 3:

| Beispiel | Ki [µm] |
|---|---|
| 3 | 3,7 |
| 5 | 0,7 |
| 7 | 1,2 |
| 8 | 4,6 |
| 9 | 1,8 |
| 10 | 0,99 |
| 12 | 0,99 |
| 13 | 1,1 |

| Beispiel | Ki [µm] |
|---|---|
| 17 | 0,39 |
| 19 | 0,55 |
| 20 | 0,36 |
| 21 | 0,71 |
| 22 | 1,3 |
| 23 | 1,4 |
| 24 | 0.46 |
| 25 | 1,3 |

Die oben beschriebenen Ergebnisse zeigen, daß die Verbindungen der allgemeinen Formel (I) bei neurodegenerativen Erkrankungen sowie Gehirnischämie verschiedener Genese eingesetzt werden können. Hierunter fallen beispielsweise: Status epileptikus, Hypoglykämie, Hypoxie, Anoxie, Gehirntrauma, Gehirnoedem, amyotrope laterale Sklerose, Huntington's Disease, Morbus Alzheimer, Hypotonie, Herzinfarkt, Hirndruck (erhöhter intrakranialer Druck), ischämischer und hämorrhagischer Stroke, globale cerebrale Ischämie bei Herzstillstand, Diabetische Polyneuropathie, Tinitus, perinatale Asphyxie, Psychose, Schizophrenie, Depression und Morbus Parkinson.

Die Verbindungen der allgemeinen Formel (I) können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, - insbesondere Lösungen zur Injektion (s.c., i.v., i.m.) und Infusion - Säfte, Emulsionen oder dispersible Pulver. Hierbei soll der Anteil der pharmazeutisch wirksamen Verbindung(en) jeweils im Bereich von 0,1 bis 90 Gew.-%, bevorzugt 0,5 bis 50 Gew.-% der Gesamtzusammensetzung liegen, d.h. in Mengen die ausreichend sind, um den unten angegebenen Dosierungsbereich zu erreichen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektions- und infusionslösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilflösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z. B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzukker) Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise parenteral - insbesondere auf dem Wege der Infusion - intravenös. Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Die Dosierung für die intravenöse Anwendung liegt bei 1 - 1000 mg pro Stunde, vorzugsweise zwischen 5 - 500 mg pro Stunde.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über der Tag zu verteilen.

Ferner können die Verbindungen der allgemeinen Formel 1 bzw. deren Säureadditionssalze auch mit andersartigen Wirkstoffen kombiniert werden.

Die nachfolgenden Beispiele illustrieren die vorliegende Erfindung ohne sie jedoch in ihrem Umfang zu beschränken:

Pharmazeutische Formulierungsbeispiele

| A) | Tabletten | pro Tablette |
|---|---|---|
| | Wirkstoff | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet , knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff | 80 mg |
| | Milchzucker | 55 mg |

(fortgesetzt)

| B) | Tabletten | pro Tablette |
|---|---|---|
| | Maisstärke<br>Mikrokristalline Cellulose<br>Polyvinylpyrrolidon<br>Natrium-carboxymethylstärke<br>Magnesiumstearat | 190 mg<br>35 mg<br>15 mg<br>23 mg<br>2 mg<br>400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.

| C) | Ampullenlösung | |
|---|---|---|
| | Wirkstoff<br>Natriumchlorid<br>Aqua pro inj. | 50 mg<br>50 mg<br>5 ml |

Der Wirkstoff wird bei Eigen-pH oder gegebenenfalls bei pH 5,5 bis 6,5 in Wasser gelöst und mit Natriumchlorid als Isotonans versetzt. die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

## Patentansprüche

1. Verbindungen der allgemeinen Formel (I)

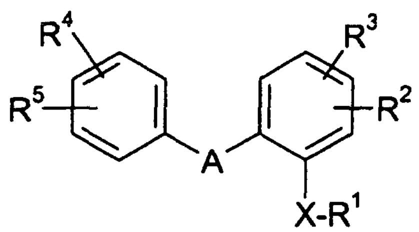

(I)

worin

A — ein Furan, Tetrahydrofuran, Dioxolan, Pyrrol, Pyrrolin, Pyrrolidin, Pyrazol, Pyrazolin, Pyrazolidin, Imidazol, Imidazolin, Imidazolidin, Triazol, Triazolin, Triazolidin, Oxazol, Oxazolin, Oxadiazol aber nicht 1,2,4-Oxadiazol, Isoxazol oder Isoxazolin, die jeweils ein- oder mehrfach durch $C_1$-$C_4$-Alkoxy, OH, =O oder $C_1$-$C_4$-Alkyl substituiert ist, wobei der $C_1$-$C_4$-Alkyl-Rest seinerseits substituiert sein kann durch Fluor, Chlor, Brom, OH oder $-NR^6R^7$;

X — Sauerstoff;

$R^1$ — ein $C_1$-$C_4$-Alkyl-Rest, der durch $-CONHSO_2R^8$, $-CONR^6R^7$, $-CH=NOR^8$, $-NR^6R^7$, $-NHCOR^8$, $-NHCONR^6R^7$, $-NHCOOR^8$, $-OCONR^6R^7$, $-SO_2NR^6R^7$ oder durch ein N-Oxid der Formel $-NOR^6R^7$ substituiert ist;

$R^2$ und $R^3$ die gleich oder verschieden sein können Wasserstoff, $-NR^6R^7$, Fluor, Chlor, Brom, Nitro, $CF_3$, CN, $-OR^8$, $C_1$-$C_4$-Alkyl, Phenyl, Benzyl oder Phenyloxy;

$R^4$ und $R^5$ die gleich oder verschieden sein können Wasserstoff, $-NR^6R^7$, Fluor, Chlor, Nitro, $CF_3$, CN, $-OR^8$, $C_1$-$C_4$-Alkyl, Phenyl, Benzyl oder Phenyloxy;

$R^6$ Wasserstoff, $C_3$-$C_6$-Cycloalkyl oder $C_1$-$C_4$-Alkyl, die jeweils ein- oder mehrfach durch Hydroxy, Phenyl, Benzyl oder $C_1$-$C_4$-Alkoxy substituiert sein können,

$R^6$ Phenyl, das gegebenenfalls durch Fluor, Chlor, Brom, $-OR^8$, $C_1$-$C_4$-Alkyl, bevorzugt $-CH_3$, $-SO_3H$, oder $-COOR^8$ substituiert sein kann;

$R^7$ Wasserstoff, $C_3$-$C_6$-Cycloalkyl oder $C_1$-$C_4$-Alkyl, die jeweils ein- oder mehrfach durch Hydroxy, Phenyl, Benzyl oder $C_1$-$C_4$-Alkoxy substituiert sein können,

$R^7$ Phenyl, das gegebenenfalls durch Fluor, Chlor, Brom, $-OR^8$, $C_1$-$C_4$-Alkyl, bevorzugt $-CH_3$, $-SO_3H$, oder $-COOR^8$ substituiert sein kann;
oder

$R^6$ und $R^7$ bilden zusammen mit dem Stickstoffatom einen gesättigten oder ungesättigten 5- oder 6-gliedrigen Ring, der als weitere Heteroatome Stickstoff oder Sauerstoff enthalten kann, wobei der Heterocyclus durch $C_1$-$C_4$-Alkyl, Phenyl oder Benzyl substituiert sein kann;

$R^8$ Wasserstoff, $C_1$-$C_4$-Alkyl, ein Benzyl- oder Phenyl-Rest, der gegebenenfalls ein- oder mehrfach durch OH, Chlor, Brom oder $OCH_3$ substituiert ist, bedeuten,
gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**2.** Verbindungen der allgemeinen Formel (I) gemäß Anspruch 1, worin

A ein Furan, Tetrahydrofuran, Dioxolan, Pyrrol, Pyrrolin, Pyrrolidin, Pyrazol, Pyrazolin, Pyrazolidin, Imidazol, Imidazolin, Imidazolidin, Triazol, Triazolin, Triazolidin, Oxazol, Oxazolin, Oxadiazol aber nicht 1,2,4-Oxadiazol, Isoxazol oder Isoxazolin, die jeweils ein- oder mehrfach durch $C_1$-$C_4$-Alkoxy, OH, =O oder $C_1$-$C_4$-Alkyl substituiert sein können, wobei der $C_1$-$C_4$-Alkyl-Rest seinerseits substituiert sein kann durch Fluor, Chlor, Brom, OH oder $-NR^6R^7$;

X Sauerstoff;

$R^1$ $C_1$-$C_4$-Alkyl, das durch $-NR^6R^7$ oder durch ein N-Oxid der Formel $-NOR^6R^7$ substituiert ist;

$R^2$ und $R^3$ die gleich oder verschieden sein können Wasserstoff, $-NR^6R^7$, Fluor, Chlor, Brom, Nitro, $CF_3$, CN, $-OR^8$, $C_1$-$C_4$-Alkyl, Phenyl, Benzyl oder Phenyloxy;

$R^4$ und $R^5$ die gleich oder verschieden sein können Wasserstoff, $-NR^6R^7$, Fluor, Chlor, Nitro, $CF_3$, CN, $-OR^8$, $C_1$-$C_4$-Alkyl, Phenyl, Benzyl oder Phenyloxy;

$R^6$ Wasserstoff oder $C_1$-$C_4$-Alkyl, das ein- oder mehrfach durch Hydroxy, Phenyl, Benzyl oder $C_1$-$C_4$-Alkoxy substituiert sein kann,

$R^7$ Wasserstoff oder $C_1$-$C_4$-Alkyl, das ein- oder mehrfach durch Hydroxy, Phenyl, Benzyl oder $C_1$-$C_4$-Alkoxy substituiert sein kann,
oder

$R^6$ und $R^7$ bilden zusammen mit dem Stickstoffatom einen gesättigten oder ungesättigten Heterocyclus ausgewählt aus der Gruppe Pyrrol, Pyrrolin, Pyrrolidin, Piperidin, Piperazin, Morpholin, Thiomorpholin, Imidazol, Imidazolin, Imidazolidin, Pyrazol, Pyrazolin, Pyrazolidin, wobei die genannten Heterocyclen gegebenenfalls durch Methyl, Ethyl, Propyl oder Benzyl substituiert sein können;

$R^8$ Wasserstoff, $C_1$-$C_4$-Alkyl, Benzyl oder Phenyl bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**3.** Verbindungen der allgemeinen Formel (I) gemäß einem der Ansprüche 1 oder 2, worin

A ein Rest ausgewählt aus der Gruppe Pyrrol, Pyrazol, Imidazol, Imidazolin, Imidazolidin, Triazol, Oxazol, Oxazolin, Isoxazol, 1,3,4-Oxadiazol oder Oxadiazolin, der gegebenenfalls ein- oder mehrfach durch OH, =O, $C_1$-$C_4$-Alkyloxy oder durch $C_1$-$C_4$-Alkyl substituiert sein kann, wobei der $C_1$-$C_4$-Alkyl-Rest seinerseits substituiert sein kann durch Chlor, Brom, OH oder $-NR^6R^7$;

X Sauerstoff;

$R^1$ $C_1$-$C_4$-Alkyl, das durch $-NR^6R^7$ substituiert ist;

$R^2$ und $R^3$ Wasserstoff;

$R^4$ und $R^5$ Wasserstoff;

$R^6$ Wasserstoff, Methyl, Ethyl, Propyl oder Butyl;

$R^7$ Wasserstoff, Methyl, Ethyl, Propyl oder Butyl;
oder

$R^6$ und $R^7$ bilden zusammen mit dem Stickstoffatom einen gesättigten oder ungesättigten Heterocyclus ausgewählt aus der Gruppe Piperidin, Piperazin oder Morpholin, die gegebenenfalls durch Methyl, Ethyl oder Benzyl substituiert sein können, bedeuten,
gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**4.** Verbindungen der allgemeinen Formel (I) gemäß einem der Ansprüche 1 bis 3, worin

A ein Rest ausgewählt aus der Gruppe Pyrrol, Pyrazol, Imidazol, Imidazolin, Imidazolidin, Triazol, Oxazol, Oxazolin, Isoxazol, 1,3,4-Oxadiazol oder Oxadiazolin, der gegebenenfalls ein- oder mehrfach durch OH, =O, $C_1$-$C_4$-Alkyloxy oder durch $C_1$-$C_4$-Alkyl substituiert sein kann, wobei der $C_1$-$C_4$-Alkyl-Rest seinerseits substituiert sein kann durch Chlor, Brom, OH oder $-NR^6R^7$;

X Sauerstoff;

$R^1$ ein Ethyl- oder Propyl-Rest, der durch $-NR^6R^7$ substituiert ist;

$R^2$ und $R^3$ Wasserstoff;

$R^4$ und $R^5$ Wasserstoff;

$R^6$ Wasserstoff, Methyl, Ethyl, Propyl oder Butyl;

$R^7$ Wasserstoff, Methyl, Ethyl, Propyl oder Butyl, bedeuten, gegebenenfalls in Form ihrer Racemate, ihrer Enantiomere, in Form ihrer Diastereomere und ihrer Gemische, sowie gegebenenfalls ihrer pharmakologisch unbedenklichen Säureadditionssalze.

**5.** Verbindungen der allgemeinen Formel (I) gemäß einem der Ansprüche 1 bis 4, worin die Gruppe

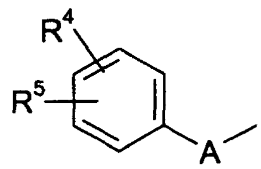

für einen der folgenden Reste stehen kann

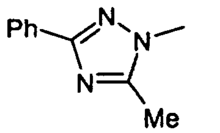
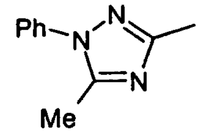
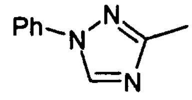
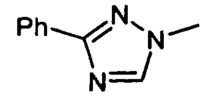
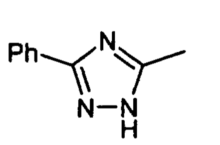
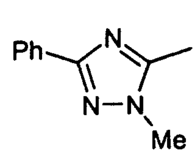
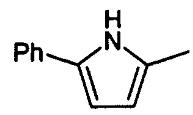
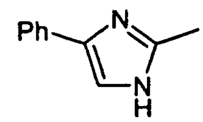
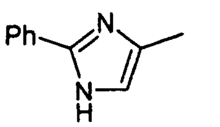
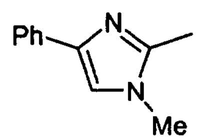
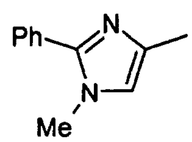
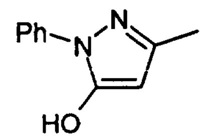
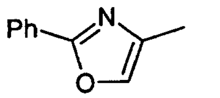
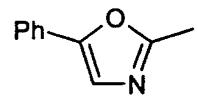
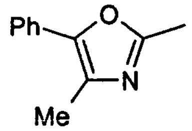
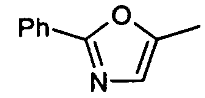
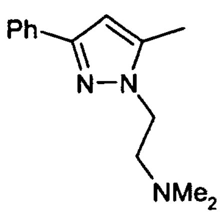
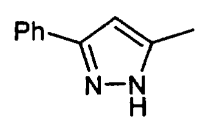
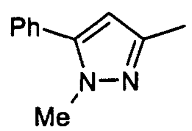
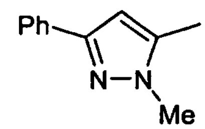
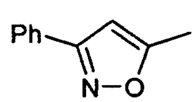
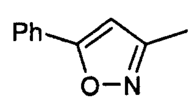
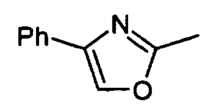
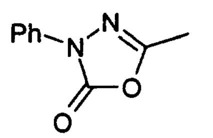
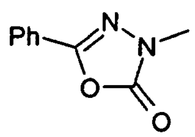
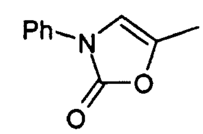
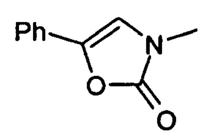
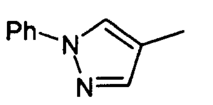
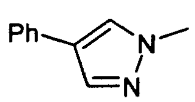
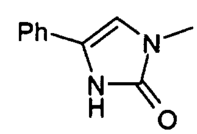
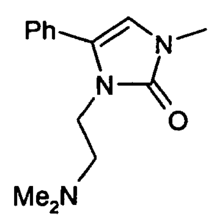
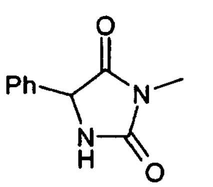
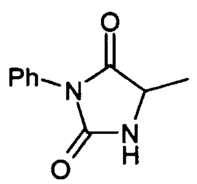
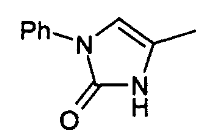
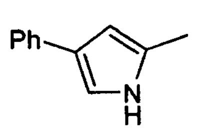
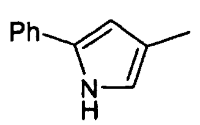
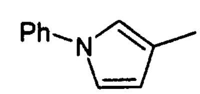
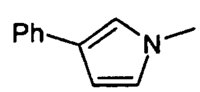

;

X Sauerstoff;

$R^1$ ein Ethyl- oder Propyl-Rest, der durch $-NR^6R^7$ substituiert ist;

R2 und $R^3$ Wasserstoff;

$R^4$ und $R^5$ Wasserstoff;

$R^6$ Wasserstoff, Methyl, Ethyl, Propyl oder Butyl;

$R^7$ Wasserstoff, Methyl, Ethyl, Propyl oder Butyl, bedeuten, gegebenenfalls in Form ihrer Racemate, ihrer Enantiomere, in Form ihrer Diastereomere und ihrer Gemische, gegebenenfalls in Form ihrer Tautomere, sowie gegebenenfalls ihrer pharmakologisch unbedenklichen Säureadditionssalze.

**6.** Verbindungen der allgemeinen Formel (I) gemäß einem der Ansprüche 1 bis 5, worin die Gruppe

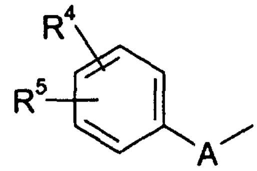

für einen der folgenden Reste stehen kann:

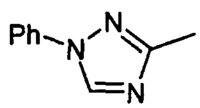
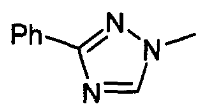
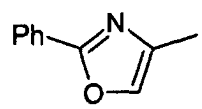
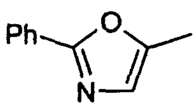
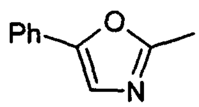
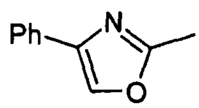
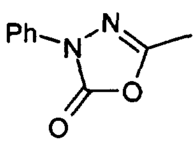
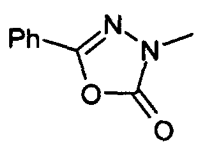
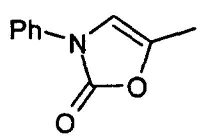
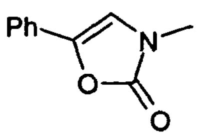
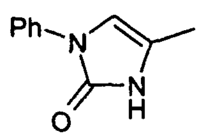
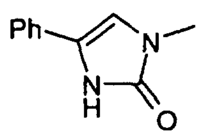
;

X Sauerstoff;

$R^1$ ein Ethyl- oder Propyl-Rest, der durch $-NR^6R^7$ substituiert ist;

$R^2$ und $R^3$ Wasserstoff;

$R^4$ und $R^5$ Wasserstoff;

$R^6$ Wasserstoff, Methyl, Ethyl, Propyl oder Butyl;

$R^7$ Wasserstoff, Methyl, Ethyl, Propyl oder Butyl, bedeuten, gegebenenfalls in Form ihrer Racemate,

ihrer Enantiomere, in Form ihrer Diastereomere und ihrer Gemische, gegebenenfalls in Form ihrer Tautomere, sowie gegebenenfalls ihrer pharmakologisch unbedenklichen Säureadditionssalze.

**7.** Verbindungen der allgemeinen Formel (I) gemäß einem der Ansprüche 1 bis 6, worin die Gruppe

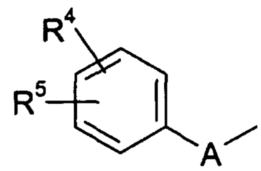

für einen der folgenden Reste stehen kann:

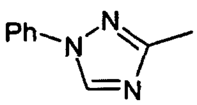

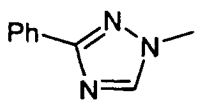

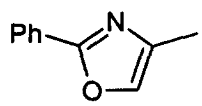

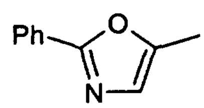

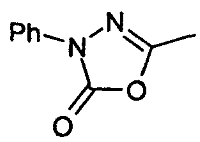

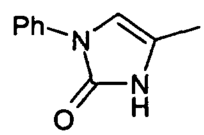

;

X Sauerstoff;

$R^1$ $-CH_2-CH_2-NR^6R^7$;

$R^2$ und $R^3$ Wasserstoff;

$R^4$ und $R^5$ Wasserstoff;

$R^6$ Methyl;

$R^7$ Methyl, bedeuten, gegebenenfalls in Form ihrer Racemate, ihrer Enantiomere, in Form ihrer Diastereomere und ihrer Gemische, gegebenenfalls in Form ihrer Tautomere, sowie gegebenenfalls ihrer pharmakologisch unbedenklichen Säureadditionssalze.

**8.** Verwendung einer Verbindung der allgemeinen Formel (I) gemäß Anspruch 1 bis 7 zur Herstellung eines Arzneimittels zur Behandlung von neurodegenerativen Erkrankungen sowie Gehirnischämie verschiedener Genese.

**9.** Pharmazeutische Zubereitungen, enthaltend als Wirkstoff eine oder mehrere Verbindungen der allgemeinen Formel (I) gemäß den Ansprüchen 1 bis 7 oder deren physiologisch verträgliche Säureadditionssalze in Kombination mit üblichen Hilfs- und/oder Trägerstoffen.

**10.** Verfahren zur Herstellung von Verbindungen der allgemeinen Formel (I)

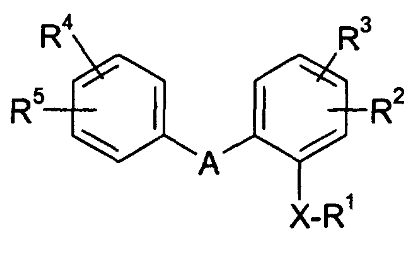

(I)

worin A, X und die Reste $R^1$, $R^2$, $R^3$, $R^4$ und $R^5$ die in den Ansprüchen 1 bis 7 genannte Bedeutung aufweisen, **dadurch gekennzeichnet, daß** eine Verbindung der allgemeinen Formel (II)

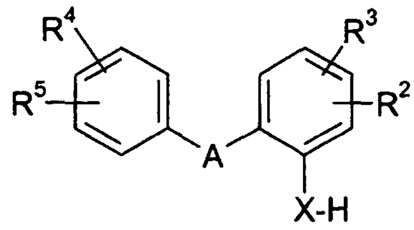

(II)

worin A, X und die Reste $R^2$, $R^3$, $R^4$ und $R^5$ die in den Ansprüchen 1 bis 7 genannte Bedeutung aufweisen, unter basischen Bedingungen mit Elektrophilen der allgemeinen Formel

$$L\text{-}R^1,$$

wobei L eine Abgangsgruppe wie beispielsweise Chlor, Brom, Iod, Methansulfonyl, Trifluormethansulfonyl oder p-Toluolsulfonyl darstellt und $R^1$ die in den Ansprüchen 1 bis 7 angegebene Bedeutung aufweist, umgesetzt wird.

## Claims

**1.** Compounds of general formula (I)

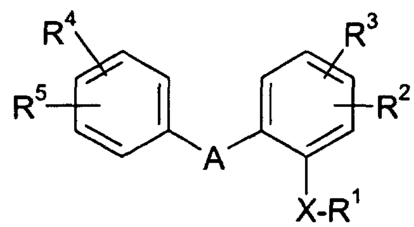

(I)

wherein

A denotes a furan, tetrahydrofuran, dioxolane, pyrrole, pyrroline, pyrrolidine, pyrazole, pyrazoline, pyrazolidine, imidazole, imidazoline, imidazolidine, triazole, triazoline, triazolidine, oxazole, oxazoline, oxadiazole but not 1,2,4-oxadiazole, isoxazole or isoxazoline, each of which may be mono- or polysubstituted by $C_1$-$C_4$-alkoxy, OH, =O or $C_1$-$C_4$-alkyl, whilst the $C_1$-$C_4$-alkyl group may in turn be substituted by fluorine, chlorine, bromine, OH or $-NR^6R^7$;

X denotes oxygen;

$R^1$ denotes a $C_1$-$C_4$-alkyl group which is substituted by $-CONHSO_2R^8$, $-CONR^6R^7$, $-CH=NOR^8$, $-NR^6R^7$, $-NHCOR^8$, $-NHCONR^6R^7$, $-NHCOOR^8$, $-OCONR^6R^7$, $-SO_2NR^6R^7$ or by an N-oxide of the formula $-NOR^6R^7$;

$R^2$ and $R^3$ which may be identical or different denote hydrogen, $-NR^6R^7$, fluorine, chlorine, bromine, nitro, $CF_3$, CN, $-OR^8$, $C_1$-$C_4$-alkyl, phenyl, benzyl or phenyloxy;

$R^4$ and $R^5$ which may be identical or different denote hydrogen, $-NR^6R^7$, fluorine, chlorine, nitro, $CF_3$, CN, $-OR^8$, $C_1$-$C_4$-alkyl, phenyl, benzyl or phenyloxy;

$R^6$ denotes hydrogen, $C_3$-$C_6$-cycloalkyl or $C_1$-$C_4$-alkyl, each of which may be mono- or polysubstituted by hydroxy, phenyl, benzyl or $C_1$-$C_4$-alkoxy,

$R^6$ denotes phenyl, which may optionally be substituted by fluorine, chlorine, bromine, -$OR^8$, $C_1$-$C_4$-alkyl, preferably -$CH_3$, -$SO_3H$ or -$COOR^8$;

$R^7$ denotes hydrogen, $C_3$-$C_6$-cycloalkyl or $C_1$-$C_4$-alkyl, each of which may be mono- or polysubstituted by hydroxy, phenyl, benzyl or $C_1$-$C_4$-alkoxy,

$R^7$ denotes phenyl, which may optionally be substituted by fluorine, chlorine, bromine, -$OR^8$, $C_1$-$C_4$-alkyl, preferably -$CH_3$, -$SO_3H$, or -$COOR^8$;
or

$R^6$ and $R^7$ together with the nitrogen atom form a saturated or unsaturated 5- or 6-membered ring which may contain nitrogen or oxygen as further heteroatoms, whilst the heterocycle may be substituted by $C_1$-$C_4$-alkyl, phenyl or benzyl;

$R^8$ denotes hydrogen, $C_1$-$C_4$-alkyl, a benzyl or phenyl group which may optionally be mono- or polysubstituted by OH, chlorine, bromine or $OCH_3$, optionally in the form of their racemates, their enantiomers, in the form of their diastereomers and the mixtures thereof, and optionally the pharmacologically acceptable acid addition salts thereof.

**2.** Compounds of general formula (I) according to claim 1, wherein

A denotes a furan, tetrahydrofuran, dioxolane, pyrrole, pyrroline, pyrrolidine, pyrazole, pyrazoline, pyrazolidine, imidazole, imidazoline, imidazolidine, triazole, triazoline, triazolidine, oxazole, oxazoline, oxadiazole but not 1,2,4-oxadiazole, isoxazole or isoxazoline, each of which may be mono- or polysubstituted by $C_1$-$C_4$-alkoxy, OH, =O or $C_1$-$C_4$-alkyl, whilst the $C_1$-$C_4$-alkyl group may in turn be substituted by fluorine, chlorine, bromine, OH or -$NR^6R^7$;

X denotes oxygen;

$R^1$ denotes $C_1$-$C_4$-alkyl which is substituted by -$NR^6R^7$ or by an N-oxide of the formula -$NOR^6R^7$;

$R^2$ and $R^3$ which may be identical or different denote hydrogen, -$NR^6R^7$, fluorine, chlorine, bromine, nitro, $CF_3$, CN, -$OR^8$, $C_1$-$C_4$-alkyl, phenyl, benzyl or phenyloxy;

$R^4$ and $R^5$ which may be identical or different denote hydrogen, -$NR^6R^7$, fluorine, chlorine, nitro, $CF_3$, CN, -$OR^8$, $C_1$-$C_4$-alkyl, phenyl, benzyl or phenyloxy;

$R^6$ denotes hydrogen or $C_1$-$C_4$-alkyl which may be mono- or polysubstituted by hydroxy, phenyl, benzyl or $C_1$-$C_4$-alkoxy,

$R^7$ denotes hydrogen or $C_1$-$C_4$-alkyl which may be mono- or polysubstituted by hydroxy, phenyl, benzyl or $C_1$-$C_4$-alkoxy, or

$R^6$ and $R^7$ together with the nitrogen atom form a saturated or unsaturated heterocycle selected from the group comprising pyrrole, pyrroline, pyrrolidine, piperidine, piperazine, morpholine, thiomorpholine, imidazole, imidazoline, imidazolidine, pyrazole, pyrazoline, pyrazolidine, whilst the abovementioned heterocycles may optionally be substituted by methyl, ethyl, propyl or benzyl;

$R^8$ denotes hydrogen, $C_1$-$C_4$-alkyl, benzyl or phenyl, optionally in the form of their racemates, their enantiomers, in the form of their diastereomers and the mixtures thereof, and optionally the pharmacologically acceptable acid addition salts thereof.

**3.** Compounds of general formula (I) according to one of claims 1 or 2, wherein

A denotes a group selected from among pyrrole, pyrazole, imidazole, imidazoline, imidazolidine, triazole, oxazole, oxazoline, isoxazole, 1,3,4-oxadiazole or oxadiazoline which may optionally be mono- or polysubstituted by OH, =O, $C_1$-$C_4$-alkyloxy or by $C_1$-$C_4$-alkyl, whilst the $C_1$-$C_4$-alkyl group may in turn be substituted by chlorine, bromine, OH or -$NR^6R^7$;

X denotes oxygen;

$R^1$ denotes $C_1$-$C_4$-alkyl which is substituted by -$NR^6R^7$;

$R^2$ and $R^3$ denote hydrogen;

$R^4$ and $R^5$ denote hydrogen;

$R^6$ denotes hydrogen, methyl, ethyl, propyl or butyl;

$R^7$ denotes hydrogen, methyl, ethyl, propyl or butyl;
or

$R^6$ and $R^7$ together with the nitrogen atom form a saturated or unsaturated heterocycle selected from the group comprising piperidine, piperazine or morpholine which may optionally be substituted by methyl, ethyl or benzyl, optionally in the form of their racemates, their enantiomers, in the form of their diastereomers and the mixtures thereof, and optionally the pharmacologically acceptable acid addition salts thereof.

**4.** Compounds of general formula (I) according to one of claims 1 to 3, wherein

A denotes a group selected from among pyrrole, pyrazole, imidazole, imidazoline, imidazolidine, triazole, oxazole, oxazoline, isoxazole, 1,3,4-oxadiazole or oxadiazoline which may optionally be mono- or polysubstituted by OH, =O, $C_1$-$C_4$-alkyloxy or by $C_1$-$C_4$-alkyl, whilst the $C_1$-$C_4$-alkyl group may in turn be substituted by chlorine, bromine, OH or -$NR^6R^7$;

X denotes oxygen;

$R^1$ denotes an ethyl or propyl group which is substituted by - $NR^6R^7$;

$R^2$ and $R^3$ denote hydrogen;

$R^4$ and $R^5$ denote hydrogen;

$R^6$ denotes hydrogen, methyl, ethyl, propyl or butyl;

$R^7$ denotes hydrogen, methyl, ethyl, propyl or butyl, optionally in the form of their racemates, their enantiomers, in the form of their diastereomers and the mixtures thereof, and optionally the pharmacologically acceptable acid addition salts thereof.

**5.** Compounds of general formula (I) according to one of claims 1 to 4, wherein the group

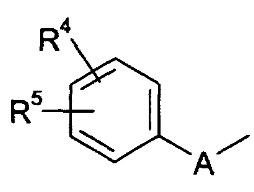

may denote one of the following groups

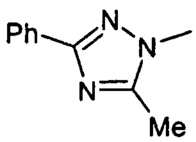
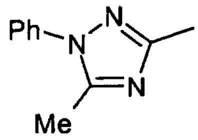
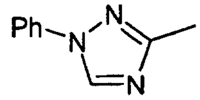
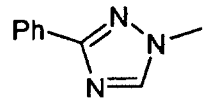
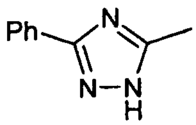
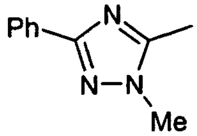
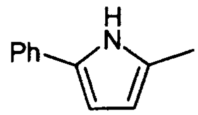
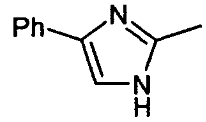
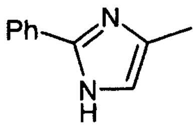
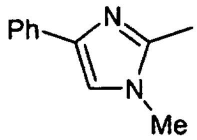
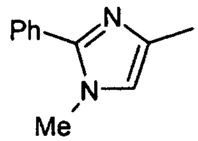
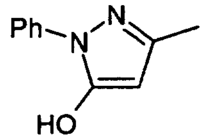
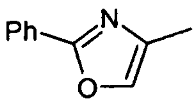
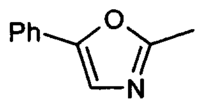
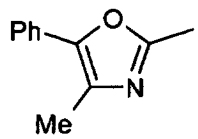
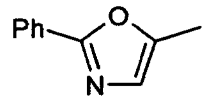
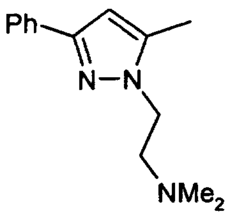
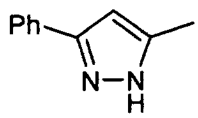
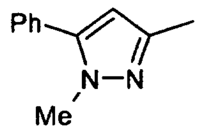
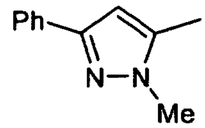
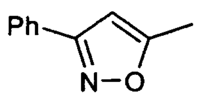
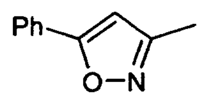
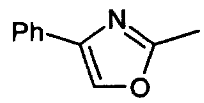
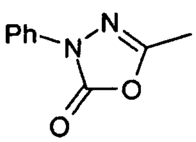
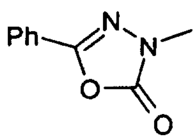
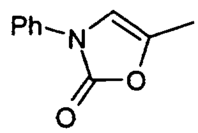
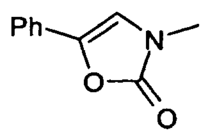
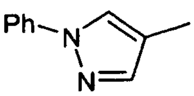
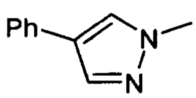
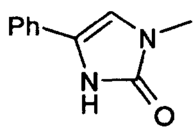
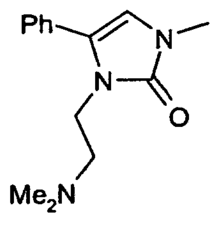
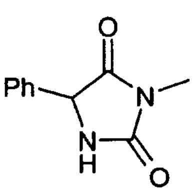
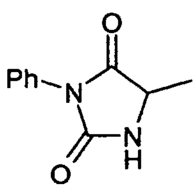
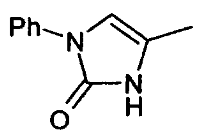
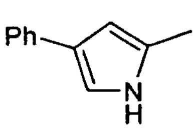
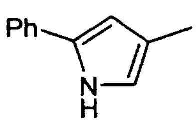
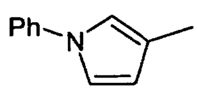
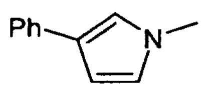

X denotes oxygen;

$R^1$ denotes an ethyl or propyl group which is substituted by $-NR^6R^7$;

$R^2$ and $R^3$ denote hydrogen;

$R^4$ and $R^5$ denote hydrogen;

$R^6$ denotes hydrogen, methyl, ethyl, propyl or butyl;

$R^7$ denotes hydrogen, methyl, ethyl, propyl or butyl, optionally in the form of their racemates, their enantiomers, in the form of their diastereomers and the mixtures thereof, optionally in the form of their tautomers, and optionally the pharmacologically acceptable acid addition salts thereof.

**6.** Compounds of general formula (I) according to one of claims 1 to 5, wherein the group

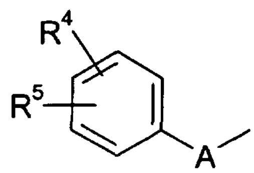

may represent one of the following groups:

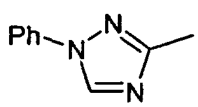
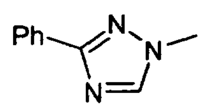
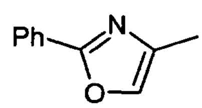
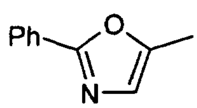
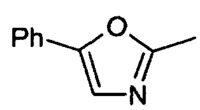
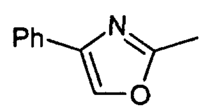
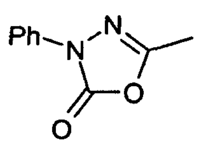
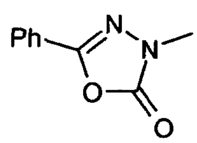
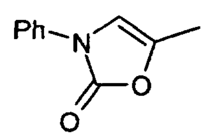
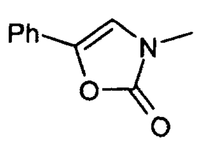
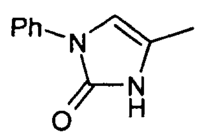
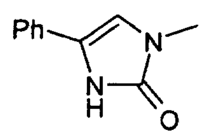

X denotes oxygen;
$R^1$ denotes an ethyl or propyl group with is substituted by $-NR^5R^7$;
$R^2$ and $R^3$ denote hydrogen;
$R^4$ and $R^5$ denote hydrogen;
$R^5$ denote hydrogen, methyl, ethyl, propyl or butyl;
$R^7$ denotes hydrogen, methyl, ethyl, propyl or butyl, optionally in the form of their racemates, their enantiomers, in the form of their diastereomers and the mixtures thereof, optionally in the form of their tautomers, and optionally the pharmacologically acceptable acid addition salts thereof.

**7.** Compounds of general formula (I) according to one of claims 1 to 6, wherein the group

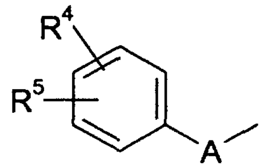

may denote one of the following groups

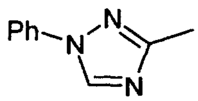
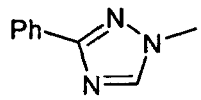
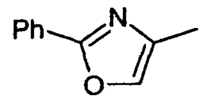
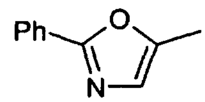
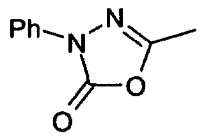
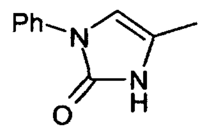
;

X denotes oxygen;

$R^1$ denotes $-CH_2-CH_2-NR^6R^7$;

$R^2$ and $R^3$ denote hydrogen;

$R^4$ and $R^5$ denote hydrogen;

$R^6$ denotes methyl;

$R^7$ denotes methyl,
optionally in the form of their racemates, their enantiomers, in the form of their diastereomers and the mixtures thereof, optionally in the form of their tautomers, and optionally the pharmacologically acceptable acid addition salts thereof.

**8.** Use of a compound of general formula (I) according to claims 1 to 7 as a pharmaceutical composition for the treatment of neurodegenerative disorders and cerebral ischaemia of various origins.

**9.** Pharmaceutical preparations containing, as active substance, one or more compounds of general formula (I) according to claims 1 to 7 or the physiologically acceptable acid addition salts thereof combined with conventional excipients and/or carriers.

**10.** Process for preparing compounds of general formula (I)

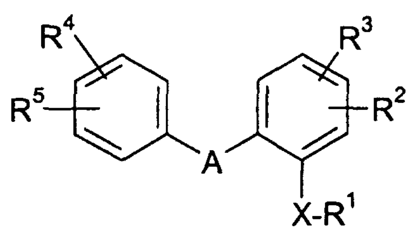
(I)

wherein A, X and the groups $R^1$, $R^2$, $R^3$, $R^4$ and $R^5$ are defined as in claims 1 to 7, **characterised in that** a compound of general formula (II)

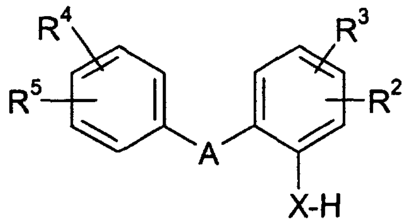

(II)

wherein A, X and the groups $R^2$, $R^3$, $R^4$ and $R^5$ are defined as in claims 1 to 7, is reacted under basic conditions with electrophiles of general formula

$$L\text{-}R^1,$$

where L is a leaving group such as, for example, chlorine, bromine, iodine, methanesulphonyl, trifluoromethanesulphonyl or p-toluenesulphonyl and $R^1$ is defined as in claims 1 to 7

## Revendications

1. Composés de formule générale (I)

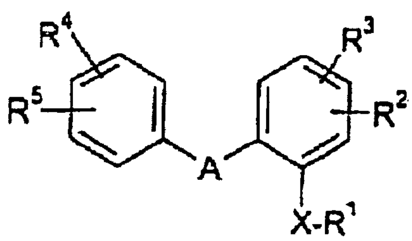

(I)

dans laquelle
A signifie un furanne, tétrahydrofuranne, dioxolane, pyrrole, pyrroline, pyrrolidine, pyrazole, pyrazoline, pyrazolidine, imidazole, imidazoline, imidazolidine, triazole, triazoline, triazolidine, oxazole, oxazoline, oxadiazole mais pas 1,2,4-oxadiazole, isoxazole ou isoxazoline, qui sont chacun substitués une ou plusieurs fois par un alcoxy en $C_1$-$C_4$, OH, =O ou alkyle en $C_1$-$C_4$, le radical alkyle en $C_1$-$C_4$ pouvant lui-même être substitué par un fluor, chlore, brome, OH ou $NR^6R^7$ ;
X signifie oxygène ;
$R^1$ signifie un radical alkyle en $C_1$-$C_4$ qui est substitué par $-CONHSO_2R^8$, $-CONR^6R^7$, $-CH=NOR^8$, $-NR^6R^7$, $-NHCOR^8$, $-NHCONR^6R^7$, $-NHCOOR^8$, $-OCONR^6R^7$, $-SO_2NR^6R^7$ ou par un oxyde d'azote de formule $-NOR^6R^7$;
$R^2$ et $R^3$ qui peuvent être identiques ou différents signifient hydrogène, $-NR^6R^7$, fluor, chlore, brome, nitro, $CF_3$, CN, $-OR^8$, alkyle en $C_1$-$C_4$, phényle, benzyle, ou phényloxy ;
$R^4$ et $R^5$ qui peuvent être identiques ou différents signifient hydrogène, $-NR^6R^7$, fluor, chlore, nitro, $CF_3$, CN, $-OR^8$, alkyle en $C_1$-$C_4$, phényle, benzyle ou phényloxy ; $R^6$ signifie hydrogène, cycloalkyle en $C_3$-$C_6$ ou alkyle en $C_1$-$C_4$ qui peuvent chacun être substitués une ou plusieurs fois par un hydroxy, phényle, benzyle ou alcoxy en $C_1$-$C_4$, $R^6$ signifie phényle, qui peut le cas échéant être substitué par un fluor, chlore, brome, $-OR^8$, alkyle en $C_1$-$C_4$, de préférence $-CH_3$, $-SO_3H$ ou $-COOR^8$ ;
$R^7$ signifie hydrogène, cycloalkyle en $C_3$-$C_6$ ou alkyle en $C_1$-$C_4$ qui peuvent chacun être substitués une ou plusieurs fois par un hydroxy, phényle, benzyle ou alcoxy en $C_1$-$C_4$, $R^7$ signifie phényle, qui peut le cas échéant être substitué par un fluor, chlore, brome, $-OR^8$, alkyle en $C_1$-$C_4$, de préférence $-CH_3$, $-SO_3H$, ou $COOR^8$;
ou
$R^6$ et $R^7$ forment ensemble avec l'atome d'azote un cycle saturé ou insaturé à 5 ou 6 atomes, qui peut contenir

de l'azote ou de l'oxygène comme hétéroatomes supplémentaires, l'hétérocycle pouvant être substitué par un alkyle en $C_1$-$C_4$, phényle ou benzyle ;
$R^8$ signifie hydrogène, alkyle en $C_1$-$C_4$, un radical benzyle ou phényle qui le cas échéant peut être substitué une ou plusieurs fois par un OH, chlore, brome ou $OCH_3$,
le cas échéant sous la formes de leurs racémiques, de leurs énantiomères, sous forme de leurs diastéréomères et de leurs mélanges, ainsi que le cas échéant de leurs sels d'addition d'acides pharmaceutiquement inoffensifs.

**2.** Composés de formule générale (I) selon la revendication 1, dans laquelle
A signifie un furanne, tétrahydrofuranne, dioxolane, pyrrole, pyrroline, pyrrolidine, pyrazole, pyrazoline, pyrazolidine, imidazole, imidazoline, imidazolidine, triazole, triazoline, triazolidine, oxazole, oxazoline, oxadiazole mais pas 1,2,4-oxadiazole, isoxazole, ou isoxazoline, qui peuvent chacun être substitués une ou plusieurs fois par un alcoxy en $C_1$-$C_4$, OH, =O, ou alkyle en $C_1$-$C_4$, le radical alkyle en $C_1$-$C_4$ pouvant lui-même être substitué par un fluor, chlore, brome, OH ou -$NR^6R^7$ ;
X signifie oxygène ;
$R^1$ signifie alkyle en $C_1$-$C_4$ qui est substitué par -$NR^6R^7$
ou par un oxyde d'azote de formule -$NOR^6R^7$;
$R^2$ et $R^3$ qui peuvent être identiques ou différents signifient hydrogène, -$NR^6R^7$, fluor, chlore, brome, nitro, $CF_3$, CN, -$OR^8$, alkyle en $C_1$-$C_4$, phényle, benzyle ou phényloxy ;
$R^4$ et $R^5$ qui peuvent être identiques ou différents signifient hydrogène, -$NR^6R^7$, fluor, chlore, nitro, $CF_3$, CN, -$OR^8$, alkyle en $C_1$-$C_4$, phényle, benzyle ou phényloxy ; $R^6$ signifie hydrogène ou alkyle en $C_1$-$C_4$ qui peut être substitué une ou plusieurs fois par un hydroxy, phényle, benzyle ou alcoxy en $C_1$-$C_4$ ;
$R^7$ signifie hydrogène ou alkyle en $C_1$-$C_4$ qui peut être substitué une ou plusieurs fois par un hydroxy, phényle, benzyle ou alcoxy en $C_1$-$C_4$ ;
ou
$R^6$ et $R^7$ forment ensemble avec l'atome d'azote un hétérocycle saturé ou insaturé choisi dans le groupe pyrrole, pyrroline, pyrrolidine, pipéridine, pipérazine, morpholine, thiomorpholine, imidazole, imidazoline, imidazolidine, pyrazole, pyrazoline, pyrazolidine, lesdits hétérocycles pouvant être substitués le cas échéant par un méthyle, éthyle, propyle ou benzyle ;
$R^8$ signifie hydrogène, alkyle en $C_1$-$C_4$, benzyle ou phényle, le cas échéant sous la forme de leurs racémiques, de leurs énantiomères, sous la forme de leurs diastéréomères et de leurs mélanges, ainsi que le cas échéant de leurs sels d'addition d'acides pharmaceutiquement inoffensifs.

**3.** Composés de formule générale (I) selon l'une des revendications 1 ou 2, dans laquelle
A signifie un radical choisi dans le groupe pyrrole, pyrazole, imidazole, imidazoline, imidazolidine, triazole, oxazole, oxazoline, isoxazole, 1,3,4-oxadiazole ou oxadiazoline, qui peut le cas échéant être substitué une
ou plusieurs fois par un OH, =O, $C_1$-$C_4$-alkyloxy ou par un alkyle en $C_1$-$C_4$, le radical alkyle en $C_1$-$C_4$ pouvant lui-même être substitué par un chlore, brome, OH ou -$NR^6R^7$;
X signifie oxygène ;
$R^1$ signifie alkyle en $C_1$-$C_4$ qui est substitué par -$NR^6R^7$ ;
$R^2$ et $R^3$ signifient hydrogène ;
$R^4$ et $R^5$ signifient hydrogène ;
$R^6$ signifie hydrogène, méthyle, éthyle, propyle ou butyle ;
$R^7$ signifie hydrogène, méthyle, éthyle, propyle ou butyle ;
ou
$R^6$ et $R^7$ forment ensemble avec l'atome d'azote un hétérocycle saturé ou insaturé choisi dans le groupe pipéridine, pipérazine ou morpholine, qui peuvent le cas échéant être substitués par un méthyle, éthyle ou benzyle, le cas échéant sous la forme de leurs racémiques, de leurs énantiomères, sous la forme de leurs diastéréomères et de leurs mélanges, ainsi que le cas échéant de leurs sels d'addition d'acides pharmaceutiquement inoffensifs.

**4.** Composés de formule générale (I) selon l'une des revendications 1 à 3, dans laquelle
A signifie un radical choisi dans le groupe pyrrole, pyrazole, imidazole, imidazoline, imidazolidine, triazole, oxazole, oxazoline, isoxazole, 1,3,4-oxadiazole ou oxadiazoline qui le cas échéant peut être substitué une ou plusieurs fois par un OH, =O, alkyloxy en $C_1$-$C_4$ ou par un alkyle en $C_1$-$C_4$, le radical alkyle en $C_1$-$C_4$ pouvant lui-même être substitué par un chlore, brome, OH
ou -$NR^6R^7$ ;
X signifie oxygène ;
$R^1$ signifie un radical éthyle ou propyle qui est substitué par -$NR^6R^7$ ;
$R^2$ et $R^3$ signifient hydrogène ;

$R^4$ et $R^5$ signifient hydrogène ;
$R^6$ signifie hydrogène, méthyle, éthyle, propyle ou butyle ;
$R^7$ signifie hydrogène, méthyle éthyle, propyle ou butyle ; le cas échéant sous la forme de leurs racémiques, leurs énantiomères, sous forme de leurs diastéréomères et de leurs mélanges, le cas échéant de leurs sels d'addition d'acides pharmaceutiquement inoffensifs.

**5.** Composés de formule générale (I) selon l'une des revendications 1 à 4, dans laquelle le groupe

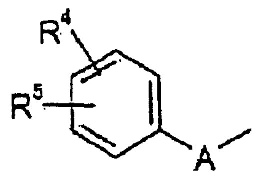

peut signifier l'un des radicaux suivants :

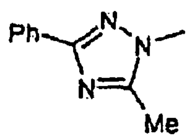 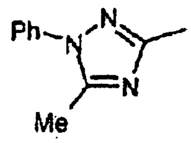 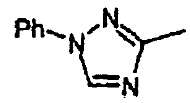 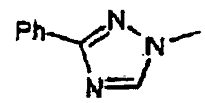

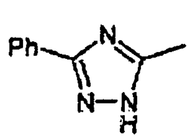 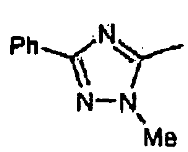 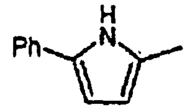 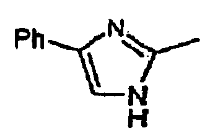

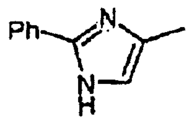 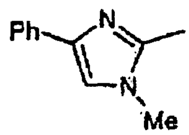 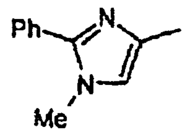 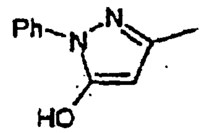

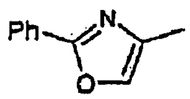 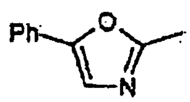 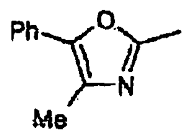 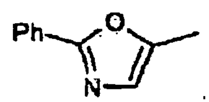

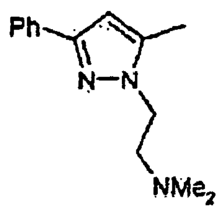 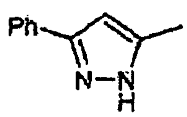 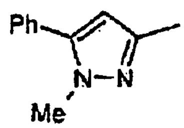 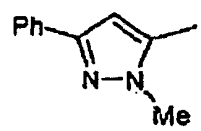

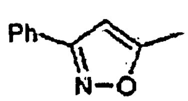 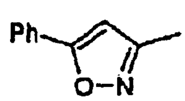 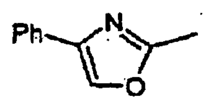

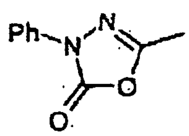 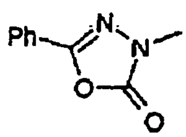 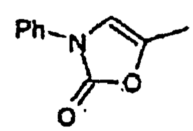 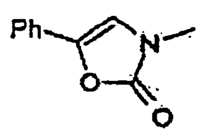

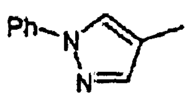 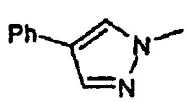 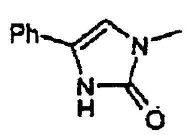 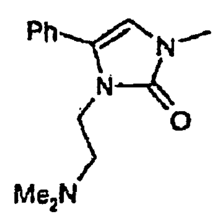

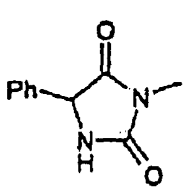 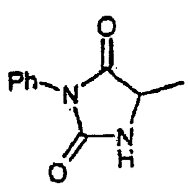 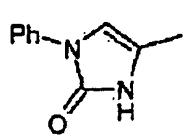

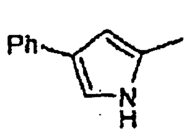 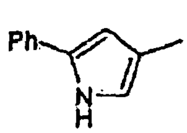 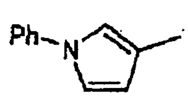 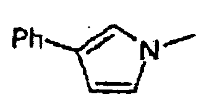

X signifie oxygène ;
$R^1$ signifie un radical éthyle ou propyle qui est substitué par $-NR^6R^7$ ;
$R^2$ et $R^3$ signifient hydrogène ;
$R^4$ et $R^5$ signifient hydrogène ;
$R^6$ signifie hydrogène, méthyle, éthyle, propyle ou butyle ;
$R^7$ signifie hydrogène, méthyle, éthyle, propyle ou butyle ;
le cas échéant sous la forme de leurs racémiques, de leurs énantiomères, sous forme de leurs diastéréomères et de leurs mélanges, le cas échéant sous forme de leurs tautomères, ainsi que le cas échéant sous forme de leurs sels d'addition d'acides pharmaceutiquement inoffensifs.

**6.** Composés de formule générale (I) selon l'une quelconque des revendications 1 à 5, dans laquelle le groupe

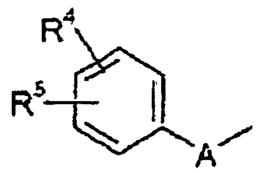

peut signifier l'un des radicaux suivants :

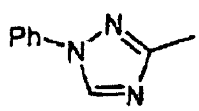 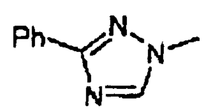 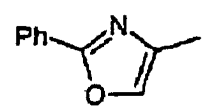

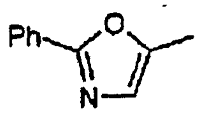 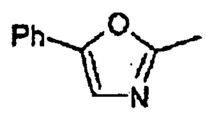 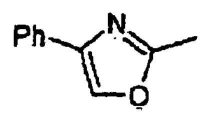

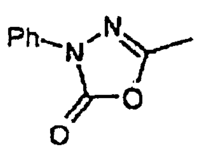 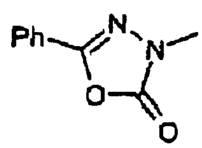 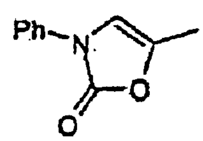

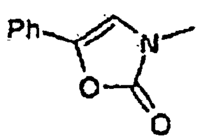 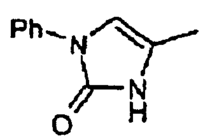 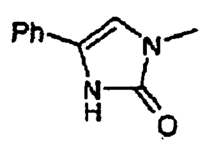

X signifie oxygène ;
$R^1$ signifie un radical éthyle ou propyle qui est substitué par $-NR^6R^7$ ;
$R^2$ et $R^3$ signifient hydrogène ;
$R^4$ et $R^5$ signifient hydrogène ;
$R^6$ signifie hydrogène, méthyle, éthyle, propyle ou butyle ;
$R^7$ signifie hydrogène, méthyle, éthyle, propyle ou butyle, le cas échéant sous la forme de leurs racémiques, de leurs énantiomères, sous forme de leurs diastéréomères et de leurs mélanges, le cas échéant sous forme de leurs tautomères, ainsi que le cas échéant sous forme de leurs sels d'addition d'acides pharmaceutiquement inoffensifs.

**7.** Composés de forme générale (I) selon l'une des revendications 1 à 6, dans laquelle le groupe

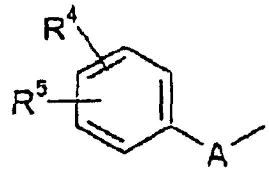

peut signifier l'un des radicaux suivants :

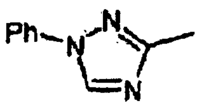
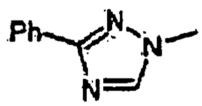
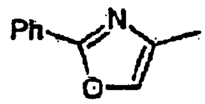
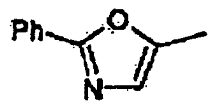

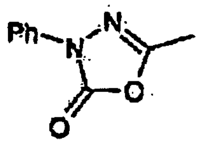
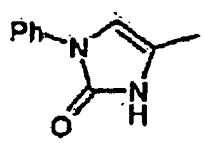
;

X signifie oxygène ;
$R^1$ signifie $-CH_3-CH_3-NR^6R^7$ ;
$R^2$ et $R^3$ signifient hydrogène ;
$R^4$ et $R^5$ signifient hydrogène ;
$R^6$ signifie méthyle ;
$R^7$ signifie méthyle,
le cas échéant sous la forme de leurs racémiques, de leurs énantiomères, sous forme de leurs diastéréomères et de leurs mélanges, le cas échéant sous forme de leurs tautomères, ainsi que le cas échéant sous forme de leurs sels d'addition d'acides pharmaceutiquement inoffensifs.

**8.** Utilisation d'un composé de formule générale (I) selon l'une des revendications 1 à 7 pour la préparation d'un médicament pour le traitement d'affections neurodégénératives et d'ischémie cérébrale d'origines diverses.

**9.** Préparations pharmaceutiques contenant comme principe actif un ou plusieurs composés de formule générale (I) selon les revendications 1 à 7 ou de leurs sels d'addition d'acides physiologiquement acceptables en combinaison avec les substances auxiliaires et de support habituelles.

**10.** Procédé pour la préparation de composés de formule générale (I)

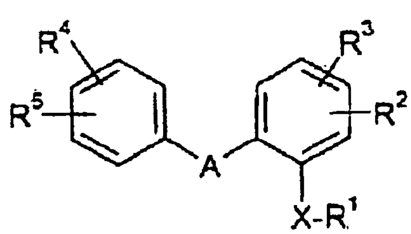
(I)

dans laquelle A, X et les radicaux $R^1$, $R^2$, $R^3$, $R^4$ et $R^5$ présentent la signification donnée dans les revendications 1 à 7, **caractérisé en ce qu'**un composé de formule générale (II)

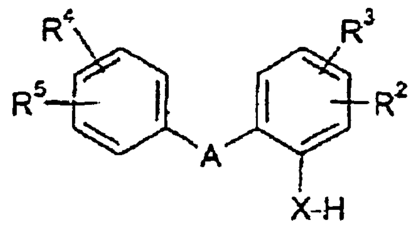

(II)

dans laquelle A, X et les radicaux $R^2$, $R^3$, $R^4$ et $R^5$ présentent la signification donnée dans les revendications 1 à 7, est mise à réagir sous des conditions basiques avec des électrophiles de formule générale

$$L\text{-}R^1$$

L étant un groupe de terminaison comme par exemple le chlore, le brome, l'iode, le méthanesulfonyle, le trifluorométhanesulfonyle ou le p-toluosulfonyle, et $R^1$ ayant la signification donnée dans les revendications 1 à 7.